# EUROPEAN PATENT APPLICATION

(11) **EP 3 906 926 A1**
(43) Date of publication of application: **10.11.2021**
(21) Application number: 20173531.3
(22) Date of filing: 07.05.2020
(51) Int. Cl.: A61K 31/472, A61K 31/50, A61K 31/513, A61K 31/5377, A61K 45/06, A61P 21/00

(54) **PROLINE HYDROXYLASE INHIBITORS FOR USE IN THE SKELETAL MUSCLE REGENERATION**

(71) Applicant: Policlinico San Donato S.p.A.- Istituto di Ricovero e Cura a Carattere Scientifico, 20097 San Donato Milanese (MI) (IT)
(72) Inventor: ANASTASIA, Luigi, I-20097 San Donato Milanese (MI) (IT); CIRILLO, Federica, I-20097 San Donato Milanese (MI) (IT)
(74) Representative: De Gregori, Antonella

(57) **Abstract**

The present invention relates to proline hydroxylase (PHDs) inhibitors acting through pharmacological activation of hypoxia inducible factor-1a (HIF-1a), upregulation of Wnt7a expression and iron chelation for use in the skeletal muscle regeneration occurring after an hypoxic insult, and related pharmaceutical compositions for administration through systemic or topical route.

SEQUENCE LISTING

<110>  POLICLINICO SAN DONATO S.p.A. -

<120>  Proline hydroxylase inhibitors for use in the skeletal muscle
       regeneration

<130>  P1850EP

<160>  36

<170>  PatentIn version 3.5

<210>  1
<211>  28
<212>  DNA
<213>  Artificial sequence

<220>
<223>  Primer forward

<400>  1
ggggtacccc ggaagctatc acaggctt                                          28


<210>  2
<211>  30
<212>  DNA
<213>  Artificial sequence

<220>
<223>  Reverse primer

<400>  2
gggttcgaac cctagcatcc ttcgctaact                                        30


<210>  3
<211>  17
<212>  DNA
<213>  Artificial sequence

<220>
<223>  WNT7a1 forward primer

<400>  3
ggaagctatc acaggct                                                      17


<210>  4
<211>  16
<212>  DNA
<213>  Artificial sequence

<220>
<223>  WNT7a1 reverse primer

<400>  4
accgtggatt caagga                                                       16


<210>  5
<211>  18

<212>  DNA
<213>  Artificial sequence

<220>
<223>  WNT7a2 forward primer

<400>  5
tgtcagcatg gctggcgc                                                    18


<210>  6
<211>  18
<212>  DNA
<213>  Artificial sequence

<220>
<223>  WNT7a2 reverse primer

<400>  6
tgtcagcatg gctggcgc                                                    18


<210>  7
<211>  19
<212>  DNA
<213>  Artificial sequence

<220>
<223>  WNT7a3 forward primer

<400>  7
gcagtctgga aggctaagc                                                   19


<210>  8
<211>  17
<212>  DNA
<213>  Artificial sequence

<220>
<223>  WNT7a3 reverse primer

<400>  8
gtcaaactca aagtcac                                                     17


<210>  9
<211>  21
<212>  DNA
<213>  Artificial sequence

<220>
<223>  WNT7a4 forward primer

<400>  9
ttcacctcct cagtttctcc a                                                21


<210>  10
<211>  20
<212>  DNA
<213>  Artificial sequence

<220>
<223>  WNT7a4 reverse primer

<400>  10
agcccaatga ccagtagcag                                                    20


<210>  11
<211>  19
<212>  DNA
<213>  Artificial sequence

<220>
<223>  WNT7a5 forward primer

<400>  11
tccagtccaa ttccaagtc                                                     19


<210>  12
<211>  18
<212>  DNA
<213>  Artificial sequence

<220>
<223>  WNT7a5 reverse primer

<400>  12
gtctgtgcga ggggctga                                                      18


<210>  13
<211>  18
<212>  DNA
<213>  Artificial sequence

<220>
<223>  WNT7a6 forward primer

<400>  13
gagggtgagg agagaatg                                                      18


<210>  14
<211>  19
<212>  DNA
<213>  Artificial sequence

<220>
<223>  WNT7a6 reverse primer

<400>  14
ccttgttaga gctctgtca                                                     19


<210>  15
<211>  18
<212>  DNA
<213>  Artificial sequence

<220>
<223>  WNT7a7 forward primer

<400>  15
cgtgagggtt ctgaaatg                                                    18


<210>  16
<211>  18
<212>  DNA
<213>  Artificial sequence

<220>
<223>  WNT7a7 reverse primer

<400>  16
ggaatcctgc ctgcctag                                                    18


<210>  17
<211>  15
<212>  DNA
<213>  Artificial sequence

<220>
<223>  VEGF forward primer

<400>  17
aaaaacgaaa gcgca                                                       15


<210>  18
<211>  15
<212>  DNA
<213>  Artificial sequence

<220>
<223>  VEGF reverse primer

<400>  18
tttctccgct ctgaa                                                       15


<210>  19
<211>  19
<212>  DNA
<213>  Artificial sequence

<220>
<223>  PHD2 forward primer

<400>  19
ctgtggaaca gcccttttt                                                   19


<210>  20
<211>  20
<212>  DNA
<213>  Artificial sequence

<220>
<223>  PHD2 reverse primer

<400>  20
cgagtctctc tgcgaatcct                                                  20

<210>  21
<211>  15
<212>  DNA
<213>  Artificial sequence

<220>
<223>  MYOG forward primer

<400>  21
agccacactg aggga                                                        15


<210>  22
<211>  15
<212>  DNA
<213>  Artificial sequence

<220>
<223>  MYOG reverse primer

<400>  22
gttgagggag ctgag                                                        15


<210>  23
<211>  20
<212>  DNA
<213>  Artificial sequence

<220>
<223>  MHC forward primer

<400>  23
tggagcagga ggaatacaag                                                   20


<210>  24
<211>  20
<212>  DNA
<213>  Artificial sequence

<220>
<223>  MHC reverse primer

<400>  24
gcatagtgga tgagggagaa                                                   20


<210>  25
<211>  17
<212>  DNA
<213>  Artificial sequence

<220>
<223>  WNT7a forward primer

<400>  25
actgtggctg cgacaag                                                      17


<210>  26
<211>  18

<212>  DNA
<213>  Artificial sequence

<220>
<223>  WNT7a reverse primer

<400>  26
cttcatgttc tcctccag                                                    18


<210>  27
<211>  18
<212>  DNA
<213>  Artificial sequence

<220>
<223>  MYOR forward primer

<400>  27
gcccagcgac atttcttc                                                    18


<210>  28
<211>  18
<212>  DNA
<213>  Artificial sequence

<220>
<223>  MYOR reverse primer

<400>  28
cgcttcctct tgcatcct                                                    18


<210>  29
<211>  17
<212>  DNA
<213>  Artificial sequence

<220>
<223>  RPL13 forward primer

<400>  29
ctcggccgtt cctgtat                                                     17


<210>  30
<211>  20
<212>  DNA
<213>  Artificial sequence

<220>
<223>  RPL13 reverse primer

<400>  30
gtggaagtgg ggcttcagta                                                  20


<210>  31
<211>  24
<212>  DNA
<213>  Artificial sequence

<220>
<223>  WNT7a target sequence

<400>  31
gggcatagtc tacctccgga tcgg                                              24


<210>  32
<211>  20
<212>  RNA
<213>  Artificial sequence

<220>
<223>  Negative control sequence

<400>  32
aaaugugaga ucagaguaau                                                   20


<210>  33
<211>  19
<212>  DNA
<213>  Artificial sequence

<220>
<223>  WNT7a forward primer

<400>  33
cttgttgcgc ttgttctcc                                                    19


<210>  34
<211>  18
<212>  DNA
<213>  Artificial sequence

<220>
<223>  WNT7a reverse primer

<400>  34
cgcaattcca cagactcg                                                     18


<210>  35
<211>  17
<212>  DNA
<213>  Artificial sequence

<220>
<223>  RPLI forward primer

<400>  35
ctcggccgtt cctgtat                                                      17


<210>  36
<211>  20
<212>  DNA
<213>  Artificial sequence

<220>
<223>  RPLI reverse primer

<400> 36
gtggaagtgg ggcttcagta 20

SEQUENCE LISTING

<110>  POLICLINICO SAN DONATO S.p.A. -

<120>  Proline hydroxylase inhibitors for use in the skeletal muscle
       regeneration

<130>  P1850EP

<160>  46

<170>  PatentIn version 3.5

<210>  1
<211>  28
<212>  DNA
<213>  Artificial sequence

<220>
<223>  Primer forward

<400>  1
ggggtacccc ggaagctatc acaggctt                                        28


<210>  2
<211>  30
<212>  DNA
<213>  Artificial sequence

<220>
<223>  Reverse primer

<400>  2
gggttcgaac cctagcatcc ttcgctaact                                      30


<210>  3
<211>  17
<212>  DNA
<213>  Artificial sequence

<220>
<223>  WNT7a1 forward primer

<400>  3
ggaagctatc acaggct                                                    17


<210>  4
<211>  16
<212>  DNA
<213>  Artificial sequence

<220>
<223>  WNT7a1 reverse primer

<400>  4
accgtggatt caagga                                                     16


<210>  5
<211>  18

<212>  DNA
<213>  Artificial sequence

<220>
<223>  WNT7a2 forward primer

<400>  5
tgtcagcatg gctggcgc                                                    18


<210>  6
<211>  18
<212>  DNA
<213>  Artificial sequence

<220>
<223>  WNT7a2 reverse primer

<400>  6
tgtcagcatg gctggcgc                                                    18


<210>  7
<211>  19
<212>  DNA
<213>  Artificial sequence

<220>
<223>  WNT7a3 forward primer

<400>  7
gcagtctgga aggctaagc                                                   19


<210>  8
<211>  17
<212>  DNA
<213>  Artificial sequence

<220>
<223>  WNT7a3 reverse primer

<400>  8
gtcaaactca aagtcac                                                     17


<210>  9
<211>  21
<212>  DNA
<213>  Artificial sequence

<220>
<223>  WNT7a4 forward primer

<400>  9
ttcacctcct cagtttctcc a                                                21


<210>  10
<211>  20
<212>  DNA
<213>  Artificial sequence

<220>
<223>  WNT7a4 reverse primer

<400>  10
agcccaatga ccagtagcag                                                    20


<210>  11
<211>  19
<212>  DNA
<213>  Artificial sequence

<220>
<223>  WNT7a5 forward primer

<400>  11
tccagtccaa ttccaagtc                                                     19


<210>  12
<211>  18
<212>  DNA
<213>  Artificial sequence

<220>
<223>  WNT7a5 reverse primer

<400>  12
gtctgtgcga ggggctga                                                      18


<210>  13
<211>  18
<212>  DNA
<213>  Artificial sequence

<220>
<223>  WNT7a6 forward primer

<400>  13
gagggtgagg agagaatg                                                      18


<210>  14
<211>  19
<212>  DNA
<213>  Artificial sequence

<220>
<223>  WNT7a6 reverse primer

<400>  14
ccttgttaga gctctgtca                                                     19


<210>  15
<211>  18
<212>  DNA
<213>  Artificial sequence

<220>
<223>  WNT7a7 forward primer

<400>  15
cgtgagggtt ctgaaatg                                                    18


<210>  16
<211>  18
<212>  DNA
<213>  Artificial sequence

<220>
<223>  WNT7a7 reverse primer

<400>  16
ggaatcctgc ctgcctag                                                    18


<210>  17
<211>  15
<212>  DNA
<213>  Artificial sequence

<220>
<223>  VEGF forward primer

<400>  17
aaaaacgaaa gcgca                                                       15


<210>  18
<211>  15
<212>  DNA
<213>  Artificial sequence

<220>
<223>  VEGF reverse primer

<400>  18
tttctccgct ctgaa                                                       15


<210>  19
<211>  19
<212>  DNA
<213>  Artificial sequence

<220>
<223>  PHD2 forward primer

<400>  19
ctgtggaaca gcccttttt                                                   19


<210>  20
<211>  20
<212>  DNA
<213>  Artificial sequence

<220>
<223>  PHD2 reverse primer

<400>  20
cgagtctctc tgcgaatcct                                                  20

<210>  21
<211>  15
<212>  DNA
<213>  Artificial sequence

<220>
<223>  MYOG forward primer

<400>  21
agccacactg aggga                                                        15


<210>  22
<211>  15
<212>  DNA
<213>  Artificial sequence

<220>
<223>  MYOG reverse primer

<400>  22
gttgagggag ctgag                                                        15


<210>  23
<211>  20
<212>  DNA
<213>  Artificial sequence

<220>
<223>  MHC forward primer

<400>  23
tggagcagga ggaatacaag                                                   20


<210>  24
<211>  20
<212>  DNA
<213>  Artificial sequence

<220>
<223>  MHC reverse primer

<400>  24
gcatagtgga tgagggagaa                                                   20


<210>  25
<211>  17
<212>  DNA
<213>  Artificial sequence

<220>
<223>  WNT7a forward primer

<400>  25
actgtggctg cgacaag                                                      17


<210>  26
<211>  18

<212>  DNA
<213>  Artificial sequence

<220>
<223>  WNT7a reverse primer

<400>  26
cttcatgttc tcctccag                                                    18


<210>  27
<211>  18
<212>  DNA
<213>  Artificial sequence

<220>
<223>  MYOR forward primer

<400>  27
gcccagcgac atttcttc                                                    18


<210>  28
<211>  18
<212>  DNA
<213>  Artificial sequence

<220>
<223>  MYOR reverse primer

<400>  28
cgcttcctct tgcatcct                                                    18


<210>  29
<211>  17
<212>  DNA
<213>  Artificial sequence

<220>
<223>  RPL13 forward primer

<400>  29
ctcggccgtt cctgtat                                                     17


<210>  30
<211>  20
<212>  DNA
<213>  Artificial sequence

<220>
<223>  RPL13 reverse primer

<400>  30
gtggaagtgg ggcttcagta                                                  20


<210>  31
<211>  24
<212>  DNA
<213>  Artificial sequence

<220>
<223>  WNT7a target sequence

<400>  31
gggcatagtc tacctccgga tcgg                                              24


<210>  32
<211>  20
<212>  RNA
<213>  Artificial sequence

<220>
<223>  Negative control sequence

<400>  32
aaaugugaga ucagaguaau                                                   20


<210>  33
<211>  19
<212>  DNA
<213>  Artificial sequence

<220>
<223>  WNT7a forward primer

<400>  33
cttgttgcgc ttgttctcc                                                    19


<210>  34
<211>  18
<212>  DNA
<213>  Artificial sequence

<220>
<223>  WNT7a reverse primer

<400>  34
cgcaattcca cagactcg                                                     18


<210>  35
<211>  17
<212>  DNA
<213>  Artificial sequence

<220>
<223>  RPLI forward primer

<400>  35
ctcggccgtt cctgtat                                                      17


<210>  36
<211>  20
<212>  DNA
<213>  Artificial sequence

<220>
<223>  RPLI reverse primer

<400>  36
gtggaagtgg ggcttcagta                                                   20


<210>  37
<211>  19
<212>  DNA
<213>  Artificial sequence

<220>
<223>  Wild type HRE binding sequence  of WNT7a promoter

<400>  37
gtctcccgtg ttccttgaa                                                    19


<210>  38
<211>  15
<212>  DNA
<213>  Artificial sequence

<220>
<223>  Deletion 1.1. of HRE sequence in the WNT7a promoter

<400>  38
gtctccttcc ttgaa                                                        15


<210>  39
<211>  17
<212>  DNA
<213>  Artificial sequence

<220>
<223>  Wild type HRE sequence of WNT7a promoter

<400>  39
tgtcgccgtg agagcgc                                                      17


<210>  40
<211>  13
<212>  DNA
<213>  Artificial sequence

<220>
<223>  Deletion 4.2 of the HRE binding sequence of WNT7a promoter

<400>  40
tgtcgcagag cgc                                                          13


<210>  41
<211>  16
<212>  DNA
<213>  Artificial sequence

<220>
<223>  Wild type of the HRE binding sequence of the WNT7a promoter

<400>  41
ttgggctgcg tgtctc                                                       16

<210>  42
<211>  12
<212>  DNA
<213>  Artificial sequence

<220>
<223>  Deletion 4.1 of the HRE binding sequence of the WNT7a promoter

<400>  42
ttgggctgtc tc                                                          12


<210>  43
<211>  17
<212>  DNA
<213>  Artificial sequence

<220>
<223>  Wild type HRE binding sequence of WNT7a pomoter

<400>  43
cctgcgcgtg ttctcag                                                     17


<210>  44
<211>  13
<212>  DNA
<213>  Artificial sequence

<220>
<223>  Deletion 4.3 of the HRE binding sequence of the WNT7a promoter

<400>  44
cctgcgttct cag                                                         13


<210>  45
<211>  50
<212>  DNA
<213>  Artificial sequence

<220>
<223>  Wild type sequence WNT7a promoter

<400>  45
gggcatagtc tacctccgga tcgggtaagc accgcgcgcg ttctttccgc                 50


<210>  46
<211>  50
<212>  DNA
<213>  Artificial sequence

<220>
<223>  KO of WNT7a promoter

<400>  46
gggcttaccc tacctccgga tggggaaggc cccgggcggt tcctttccgc                 50

## Description

The present invention relates to proline hydroxylase (PHDs) inhibitors acting through pharmacological activation of hypoxia inducible factor-1α (HIF-1α), upregulation of WNT7a expression and iron chelation for use in the skeletal muscle regeneration occurring after an hypoxic insult, and related pharmaceutical compositions for administration through systemic or topical route.

Skeletal muscle regeneration is a complex process which occurs throughout the lifespan thank to an adult stem cell reservoir present in the adult muscle. In particular, the satellite cell population (about 1% of the muscle cell population) is a 9:1 mix of Pax7⁺/Myf5⁺ muscle committed progenitors, that can be triggered to differentiate and regenerate the tissue, and Pax7⁺/Myf5⁻ uncommitted *bona fide* adult stem cells, that keep replenishing the committed pool [1]. Many factors, including aging, can reduce or damage this stem cell population, altering muscle homeostasis and causing degeneration [2]. Attempts to increase regeneration by inducing muscle differentiation ultimately failed, as they triggered a rapid depletion of the stem cell reservoir [3]. On the other hand, the cell-secreted factor Wnt7a has been shown to stimulate symmetric satellite stem cell division, dramatically enhancing muscle regeneration, whereas its depletion caused a marked reduction in the number of satellite cells following muscle healing [4]. Wnt7a is a member of the Wnt signaling, which is crucial during embryonic development and tissue homeostasis [5]. In particular, Wnt7a has been revealed to be essential for muscle differentiation, as it promotes skeletal muscle hypertrophy [6]). Indeed, Wnt7a over-expression or treatment with a Wnt7a recombinant protein has been shown to stimulate myoblasts to form hypertrophic myotubes ([6]-[8]). Moreover, treatment with exogenous Wnt7a can affect myogenesis, as it increases satellite cells motility and engraftment, resulting in a muscle strength enhancement [7]. Despite efforts have been made to therapeutically exploit Wnt7a, little is known about its upstream regulators [8].

While attempts to therapeutically use of Wnt7a have faced the high hydrophobicity of its protein, the activation of HIF-1α has been already shown to be feasible, also in humans, and several clinical trials for other pathologies are currently in advanced phases ([9], [10]). The effects of HIF-1α activation have been extensively studied in several diseases, including cancer, as it was shown to promote neo-angiogenesis and cell survival ([11],[12]). However, previous reports failed to recognize its direct contribution to the myogenic machinery and even created the misconception that its activation would primarily impair the process ([13], [14]). Ultimately, results showed that the hypoxia inducible factors (HIFs), which are induced under the hypoxic conditions typical of satellite cell niches, contribute to preserving muscle progenitor cells in a quiescent, undifferentiated state ([13], [15]-[18]). Along this line, Di Carlo et al. [13] reported the first evidence that exposure to prolonged hypoxia blocks myogenesis, and inhibited MyoD, Myf5, and myogenin expression. More recently, it was shown that hypoxic culture conditions favor the quiescence of satellite cell-derived primary myoblasts by upregulating Pax7, a key regulator of satellite cell self-renewal, and downregulating MyoD and Myogenin ([19]). Overall, these and other studies corroborated the notion that hypoxia is detrimental for muscle differentiation and HIFs activation was acknowledged to inhibit the process [14]. However, evidence claim that the role of each of the three different HIFs (HIF-1α, HIF-2α, and HIF-3α) is distinct, and their activation is dependent on the degree and the duration of oxygen deprivation ([20-22]). Lately, it was established that, among all different HIFs, only HIF-2α is responsible for maintaining satellite cells in a quiescent state [23]. HIF-2α is activated under mild hypoxia (2-9%, consistent with the oxygen tension of the stem cell niche), whereas HIF-1α is induced only under extreme hypoxia (below 1% O₂), such as in the case of an ischemic event [23].

A previous paper of the authors of the invention compelled evidence that both HIF-1α activation and the Wnt non-canonical pathway were necessary to induce myogenesis and cause the formation of hypertrophic myotubes upon transient hypoxic preconditioning (up to 24 hours at 1% O₂) [24]. However, the authors failed to recognize the direct molecular link between HIF-1α and Wnt7a, and thus could not rule out that the effects could have been indirect.

Now the authors of the present invention discovered that during physical and chemical hypoxia, WNT7a is a direct target of the hypoxia inducible factor-1α (HIF-1α) in skeletal muscle myoblasts, as it regulates WNT7a gene expression by specifically binding to two distinct Hypoxia Response Elements (HREs) on its promoter, supporting HIF-1α as a novel WNT7a activator.

In particular, *ChiP* experiments revealed the direct binding of HIF-1α to the WNT7a promoter. Moreover, site-directed mutagenesis experiments identified two distinct Hypoxia Response Elements (HREs) on the WNT7a promoter, which were specifically recognized by HIF-1α and previously unknown.

With these premise in mind the authors surprisingly discovered that chemical activation of HIF-1α through PHDs inhibitors induced the myogenesis *in vitro,* promoting the formation of hypertrophic myotubes although their total number unchanged. Particularly, at the molecular level, treatment with PHDs inhibitors activated MyoD nuclear translocation even before subjecting myoblasts to differentiation, supporting that the drug increased their commitment to myogenesis.

The effects of the PHDs inhibitors were tested in a mouse model of muscle regeneration after CTX-induced injury. For the in vivo experiments, FG-4592 was chosen, as the compound is safe for human treatment [9]. Results supported that FG-4592 treatment improved muscle regeneration after CTX-induced muscle injury, as it led to the formation of significantly bigger fibers. Remarkably, the effects appeared to be independent of angiogenesis activation, as no changes in the endothelial marker CD31 expression could be observed.

It is an object of the present invention a proline hydroxylase inhibitor activating hypoxia inducible factor-1α (HIF-1α),upregulating WNT7a expression and chelating iron, for use in the skeletal muscle regeneration. Preferably, said skeletal muscle regeneration occurs after physical or chemical hypoxic insult.

According to a preferred embodiment of the invention the proline hydroxylase inhibitor of the invention is intended for use in the treatment of muscular atrophy, sarcopenia, myopathies and muscular distrophy.

In a preferred embodiment of the present invention said proline hydroxylase inhibitor is selected among the following groups:
i) an α-ketoglutarate analogue selected from the group consisting of: 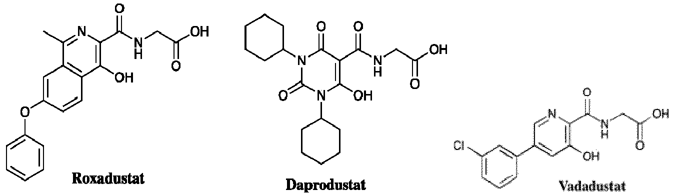 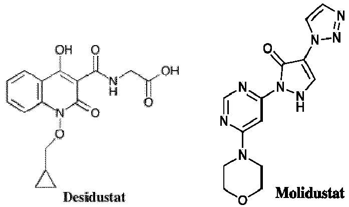 their esters or salts;
ii) a substituted diacyl-hydrazine compound characterized by the general formula (I): 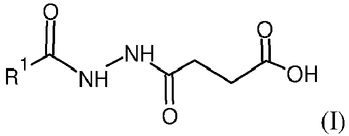 wherein R₁ is selected between isoquinoline 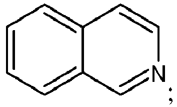 and a nitrogen aromatic ring of formula (II): 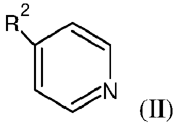
   wherein R₂ is an amine alkyl group selected from the group consisting of NHR₃ NR₃R₄, wherein R₃ and R₄ ugual or different each other, represents a linear or branched C1-C4 alkyl group;
   or R₂, jointly with the C atom to which it is bound, forms a condensed monocycle or bicycle with 3-8 terminals, which contains from 1 to 2 nitrogen atoms, said condensed cycle being selected from the group consisting of: 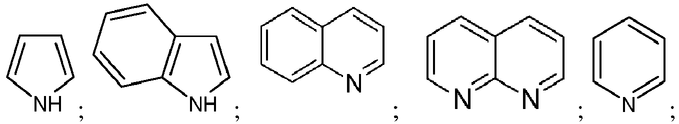 their esters or salts.

Preferably, the esters of the are linear or branched C₁-C₄ alkyl esters. More preferably they are linear alkyl ester selected between methyl, ethyl, propyl and butyl ester.

According to a preferred embodiment of the invention the analogue of α-ketoglutarate ii) is Roxadustat, and esters or salts thereof.

In a preferred embodiment of the invention the substituted diacyl-hydrazine compound of formula (I) is selected from the group consisting of: 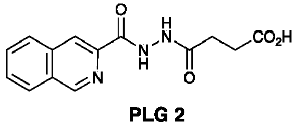 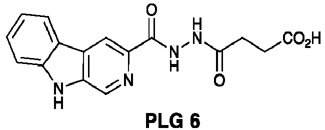 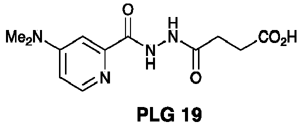 and the corresponding methyl esters thereof:

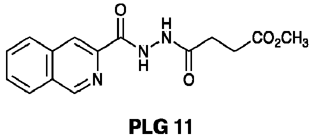

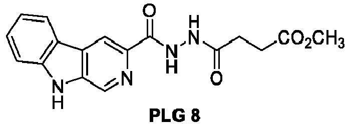

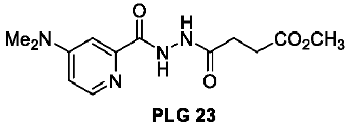

The diacyl-hydrazine residue of the inhibitors of formula I is the same function of the glycinic residue of the α-ketoglutarate analogues, that is to mime 2OG (α-ketoglutarate).

The above group i) an ii) of inhibitors of proline hydroxylase are characterized by the fact that they act as iron chelating agents, in particular of Fe(II). In particular the substituted diacyl-hydrazine compounds of formula (I) are additionally able to interact with a second iron by molecular rearrangement, thus inhibiting PHDs. The terminal carboxylic moiety which is present in both the inhibitors interacts with Arg383 of the hydroxylase.

Therefore, the inhibitors of the invention are characterized by a double mechanism of action, as they reduce the availability of the cellular iron in the cells and act directly on hypoxia inducible factor-1α (HIF-1α),finally upregulating WNT7a expression. Esters or salts are possible derivatives of the above proline hydroxylase inhibitor being acids characterized by a free carboxylic terminal. Such derivatives may improve the membrane permeability acting as a prodrug. In a preferred embodiment, the inhibitors of the invention for use in the treatment of muscular atrophy, sarcopenia, myopathies and muscular distrophy may be esterified with linear or branched C1-C6 alkyl chains selected from methyl, ethyl, propyl, isopropyl, π-butyl, sec-butyl, isobutyl, tert-butyl and pentyl. Additionally, (7-hydroxy-2-oxo-2H-1-benzopyran-4-yl)methyl ester or [2-oxo-7-(1-pyrrolidinyl)-2H-1-benzopyran-4-yl]methyl ester can be used.

Salts of the proline hydroxylase inhibitors of the invention may comprise acid addition salts. Representative salts may include acetate, arginine, benzenesulfonate, benzoate, bicarbonate, bisulfate, bitartrate, borate, bromide, calcium edetate, camsylate, carbonate, chloride, clavulanate, choline, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, lysine, malate, maleate, mandelate, meglumine, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, sulfate, tannate, tartrate, teoclate, tosylate, triethiodide, trimethylammonium, valerate salts, N,N'-dibenzylethylene diammonium, iron(III), iron(II), calcium, L-proline salt, nicotinamide salt, 1,4-dioxane salt, acetic acid salt, 2-naphthalenesulfonic acid salt, caffeine salt, tert-butylamine salt, diethylamine salt, dicyclohexylamine salt, ammonia salt, lithium salt, 3-ethyl-1-methyl-1H-imidazol-3-ium salt, trifluoroacetic acid salt, alluminium, magnesium, complex with 1,1,1,3,3,3-hexafluoro-2-propano, 2-aminoethanol, 2,2'-iminobis[ethanol], 2-amino-2-(hydroxymethyl)-1,3-propanediol, 3-phenyl-2-propenamide, dipiperazine salt, 1,2-diisopropyl-4,4,5,5-tetramethylbiguanidium salt, tromethamine salt, L-arginine salt, bis(ethylenediamine) complex, silver(I), piperazine complex, 1,2-diisopropyl-1-methyl-4,4,5,5-tetramethylbiguanidium salt, guanidinium, choline, choline chloride, tromethamine salt, rac-ketamine. The salts may be in crystalline or amorphous forms.

In a further aspect of the invention the proline hydroxylase inhibitor for use in the treatment of muscle atrophy or to promote skeletal muscle regeneration may be advantageously administered by systemic or topical route.

According to a further aspect of the present invention the proline hydroxylase inhibitors for use in the treatment of muscular atrophy, sarcopenia, myopathies and muscular distrophy may be used in a dosage range between 0.5 mg/kg and 200 mg/kg, preferably between 1 mg/kg to 100 mg/kg, more preferably between 2 mg/kg to 70 mg/kg.

The invention further contemplates a pharmaceutical composition comprising a proline hydroxylase inhibitor of the invention, together with pharmaceutically acceptable additives and/or excipients, for use in the skeletal muscle regeneration. Preferably, said skeletal muscle regeneration occurs after physical or chemical hypoxic insult.

According to a preferred embodiment the pharmaceutical composition of the invention is intended for use in the treatment of muscle atrophy, sarcopenia, myopathies and muscular distrophy.

In a preferred embodiment of the present invention the pharmaceutical composition of the invention comprises a proline hydroxylase inhibitor selected among the following groups:
i) an α-ketoglutarate analogue selected from the group consisting of: 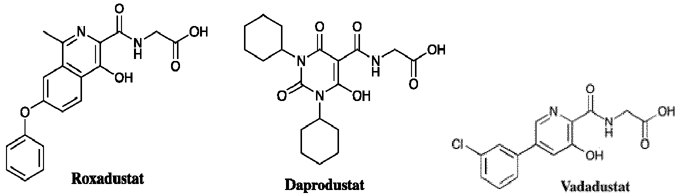 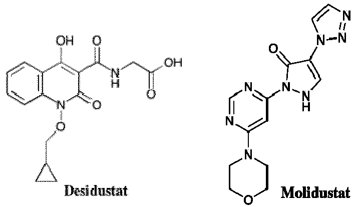 their esters or salts;
ii) a substituted diacyl-hydrazines compound characterized by general formula (I): 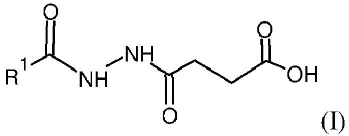 wherein R₁ is selected between isoquinoline 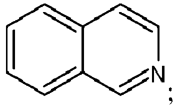 and a nitrogen aromatic ring of formula (II): 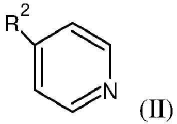 wherein R₂ is selected between:
   - an amine alkyl group selected from the group consisting of NHR₃, NR₃R₄, wherein R₃ and R₄ ugual or different each other, represents a linear or branched C₁-C₄ alkyl group;
   - an eteroaryl group selected from the group consisting of: 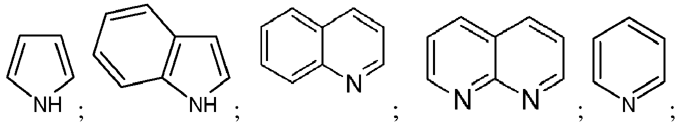 their esters or salts. Preferably, the esters of the are linear or branched C₁-C4 alkyl esters. More preferably they are linear alkyl ester selected between methyl, ethyl, propyl and butyl ester.

According to a preferred embodiment of the invention the analogue of α-ketoglutarate ii) is Roxadustat, and esters or salts thereof.

In a preferred embodiment of the invention the substituted diacyl-hydrazine compound ii) is selected from the group consisting of: 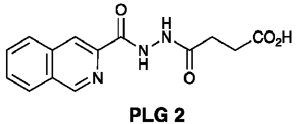 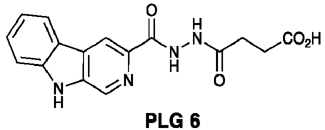 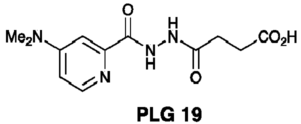 and the corresponding methyl esters thereof:

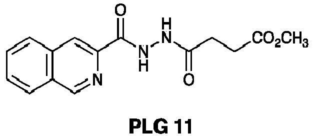

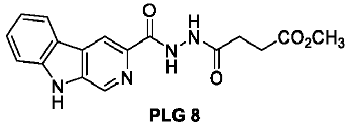

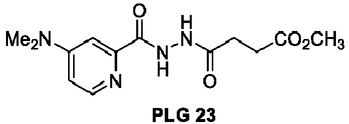

According to a particular embodiment the pharmaceutical composition of the invention are intended for systemic or topical administration. Suitable routes include oral (including buccal and sublingual), parenteral (including subcutaneous, intramuscular, intravenous, intradermal, intrathecal, and epidural and intraperitoneal), transdermal, topical, vaginal, rectal, inhalation, ophthalmic. Typical pharmaceutical formulations to be administered by oral route contemplate capsules, powders, granules, granulate, pills, suppositiers, tablets, solutions or suspensions in aqueous or non-aqueous liquids and emulsions.

According to an alternative approach the pharmaceutical composition are administered locally. Topical route may contemplate pharmaceutical forms such as gel, cream, lotions, sprays, oils, ointments, pastes, foams. Additionally, transdermal patch (multi or single-layer), reservoir or matrix systems can be used for topical administration. Various delivery systems e.g. encapsulation in liposome, microparticles, microcapsules or capsules may be advantageously used to administer topically the pharmaceutical composition of the invention.

The present invention will now be described, for non-limiting illustrative purposes, according to a preferred embodiment thereof, with particular reference to the attached figures, wherein:

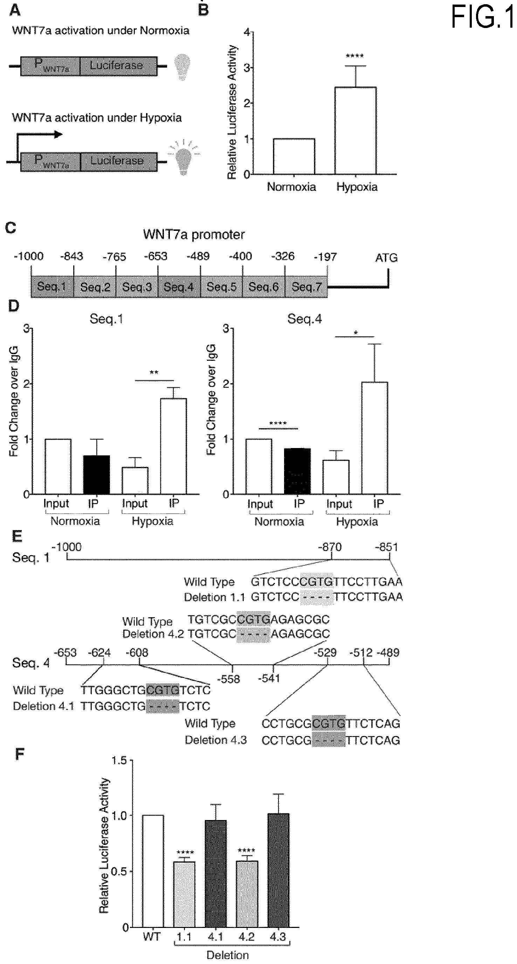

- Figure 1 shows the assessment of HIF-1α binding to the WNT7a promoter. Panel A) Schematic representation of pNL1.1 plasmid containing the WNT7a promoter upstream of the luciferase gene reporter. Under normoxia, HIF-1α is degraded and cannot bind the HRE sequences on WNT7a promoter, inhibiting the luminescence signal; under hypoxia, HIF-1α is stabilized and can bind WNT7a promoter inducing the expression of the luciferase gene. Panel B) Quantification of pNL1.1-WNT7a promoter activity by luciferase assay in murine myoblasts under normoxia and hypoxia (1% O₂). Panel C) Schematic representation of the seven binding sequences designed within the WNT7a promoter and used for qPCR analysis after Chromatin Immunoprecipitation. Panel D) qPCR analysis of HIF-1α binding on Seq. 1 and Seq. 4 of the WNT7a promoter. Panel E) Schematic representation of the deletions of the HRE binding sequences identified in Seq. 1 (Deletion 1.1) and Seq. 4 (Deletion 4.1, 4.2, and 4.3) inserted in the pNL1.1-WNT7a promoter construct using site-directed mutagenesis experiments. Panel F) Quantification of mutated pNL1.1-WNT7a promoter activity by luciferase assay. Data information: all data represent mean±SEM of three independent experiments for the Chromatin Immunoprecipitation and five independent experiments for all the other assays and the statistical analysis was determined by Student's t-test. *p < 0.05, **p < 0.01, ****p<0.0001.

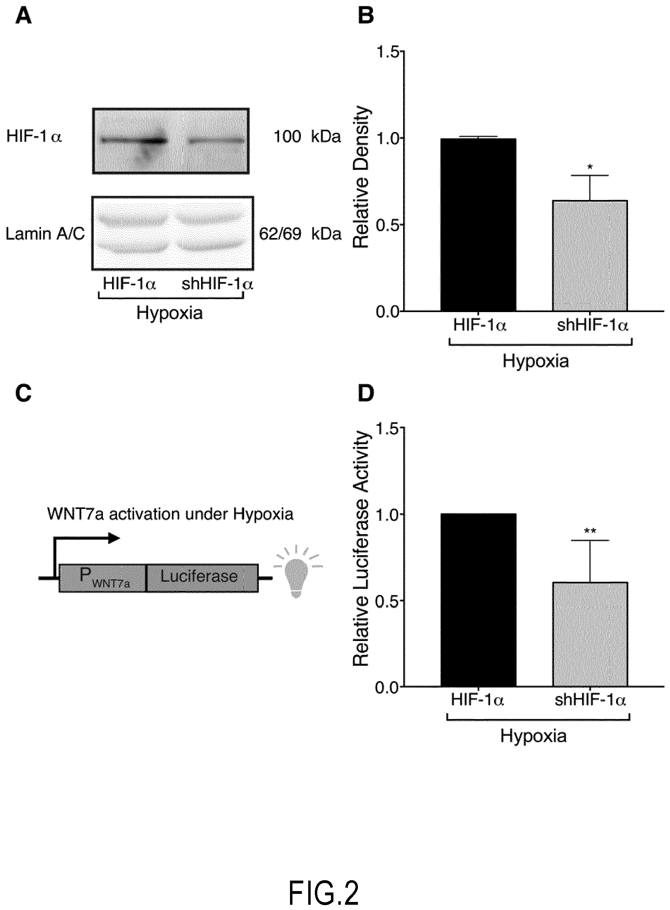

- Figure 2 shows a characterization of WNT7a promoter activity in HIF-1α-silenced myoblasts. Panels A, B) Analysis of HIF-1α nuclear translocation in HIF-1α-silenced (shHIF-1α and wild-type (HIF-1α) murine myoblasts by Western Blot. The nuclear marker Lamin A/C was used as the housekeeper. Panel C) Schematic representation of pNL1.1 plasmid containing the WNT7a promoter upstream of the luciferase gene reporter. Panel D) Quantification of pNL1.1-WNT7a promoter activity by luciferase assay in HIF-1α-silenced (shHIF-1α), as compared to wild-type cells (HIF-1α). Data information: all data represent mean ±SEM of three independent experiments and the statistical analysis was determined by Student's t-test (*p<0.05, **p<0.01).

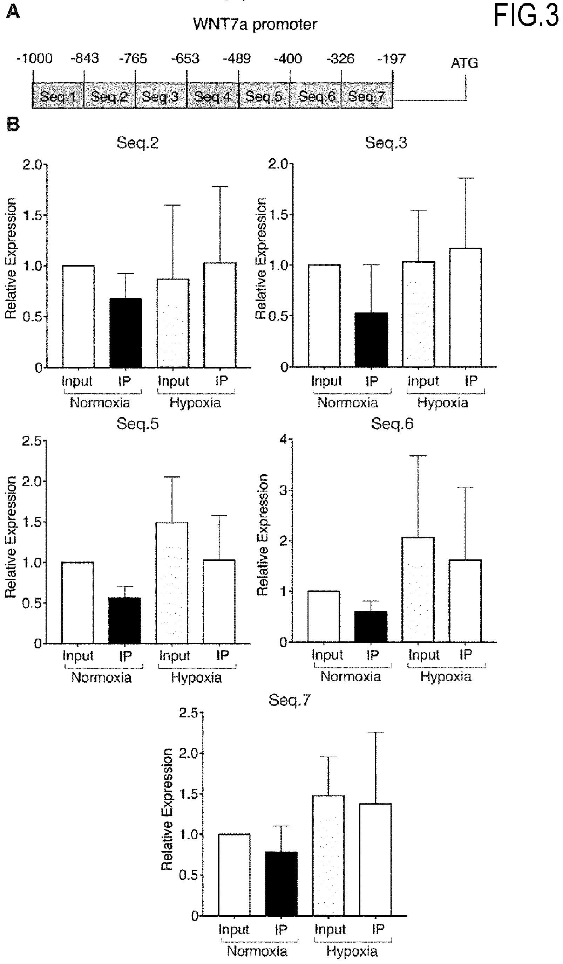

- Figure 3 shows sequences of WNT7a promoter not involved in HIF-1α binding. Panel A) Schematic representation of WNT7a promoter divided in seven binding sequences amplified by qPCR after Chromatin Immunoprecipitation. Panel B) Real-time Quantitative PCR of Seq.2, Seq.3, Seq.5, Seq.6 and Seq.7 not implicated in the binding of HIF-1α on WNT7a promoter. Data information: all data represent mean ±SEM of three independent experiments for the Chromatin Immunoprecipitation and the statistical analysis was determined by Student's t-test showing no significant differences (*p > 0.05).

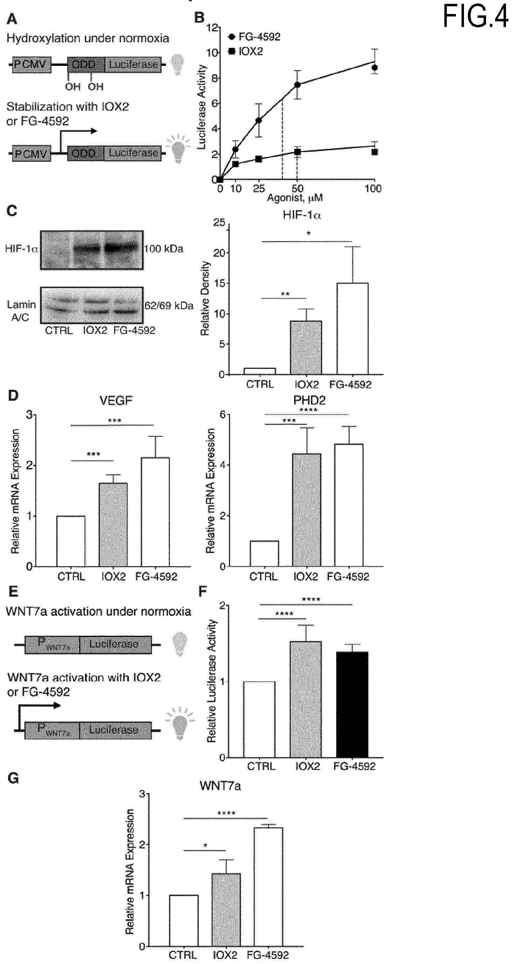

- Figure 4 shows pharmacological activation of HIF-1α with PHDs inhibitors and its effects on WNT7a regulation. Panel A) Schematic representation of the pcDNA3 construct containing the ODD-luciferase reporter gene. Under normoxia, the proline residues on the ODD sequence are hydroxylated by the PHDs, and the complex degraded by the proteasome, eventually inhibiting the generation of the luminescence signal; the pharmacological inhibition of the PHDs causes the stabilization of the ODD inducing the production of luminescence. Panel B) IC₅₀ quantification of IOX2 or FG-4592 by luciferase assay with the ODD-pcDNA3 construct. Panel C) Western blot analysis and relative quantification of HIF-1α nuclear localization in murine myoblasts treated with IOX2 or FG-4592 for 24 hours, the nuclear marker Lamin A/C was used as the housekeeper. Panel D) qPCR analysis of HIF-1α target genes, VEGF and PHD2, upon PHDs inhibition with IOX2 or FG-4592. Panel E) Schematic representation of pNL1.1 plasmid containing the WNT7a promoter upstream of the luciferase gene reporter under normoxia and upon IOX2 or FG-4592 treatment. Panel F) Quantification of pNL1.1-WNT7a promoter activity by luciferase assay of murine myoblasts treated with IOX2 or FG-4592. Panel G) qPCR analysis of WNT7a gene expression upon IOX2 or FG-4592 treatment. Data information: all data represent mean±SEM of three independent experiments and the statistical analysis was determined by Student's t-test. *p < 0.05, **p < 0.01, ***p<0.001, ****p<0.0001.

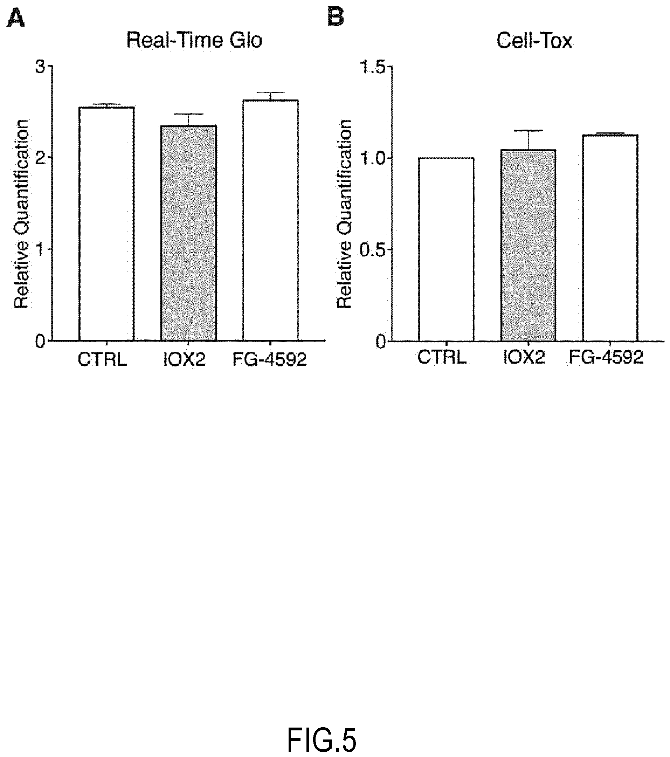

- Figure 5 shows analysis of murine myoblasts viability upon PHDs chemical treatment. Panel A) Cell viability analyzed by with RealTime-Glo™ kits MT Cell Viability Assay. Panel B) Cell toxicity measured with CellTox™ Cytotoxicity Assay. Data information: all data represent mean ±SEM of three independent experiments and the statistical analysis was determined by Student's t-test showing no significant differences (*p > 0.05).

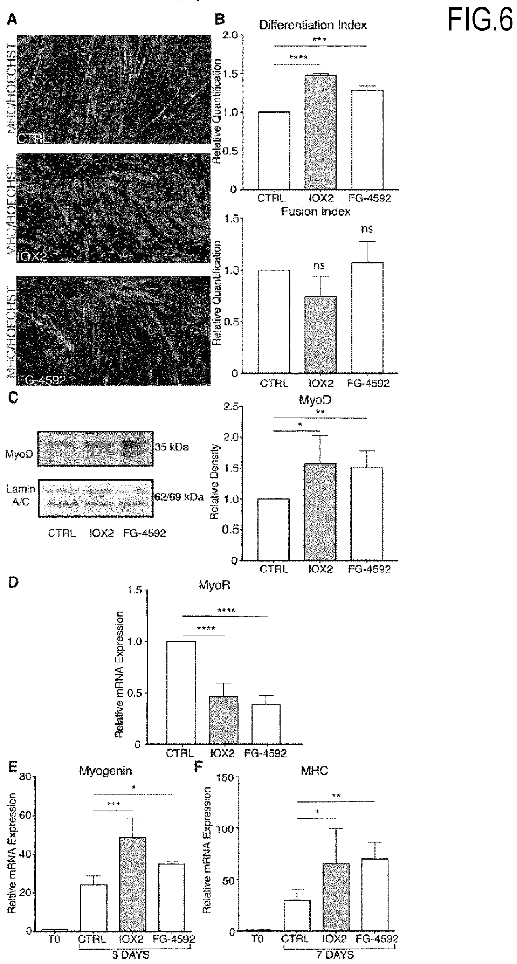

- Figure 6 shows the effects of HIF-1α pharmacological activation on skeletal muscle differentiation. Panel A) Immunofluorescence staining of MHC (green) in murine myoblasts treated with IOX2 or FG-4592 for 24 hours in GM and then switched to DM without the PHDs inhibitors for 7 days. Nuclei were stained with Hoechst 33342. Magnification is 200x. Panel B) Quantification of the fusion index, as the ratio between MHC-positive nuclei and the total number of nuclei, and of the differentiation index, as the ratio between myotubes area and MHC-positive nuclei at the end of differentiation. Panel C) Western blot analysis and relative quantification of MyoD nuclear localization of murine myoblasts treated for 24 hours with IOX2 or FG-4592, the nuclear marker Lamin A/C was used as the housekeeper. Panel D) qPCR analysis of MyoR upon PHDs inhibitors treatment. Panels E, F) qPCR analyses of Myogenin, at three days and of MHC at seven days of differentiation, in murine myoblasts pre-treated with IOX2 or FG-4592. Data information: all data represent mean±SEM of four independent experiments and the results were expressed as fold-change in comparison to normoxia. The statistical analysis was determined by Student's t-test. *p < 0.05, **p < 0.01, ***p<0.001, ****p<0.0001.

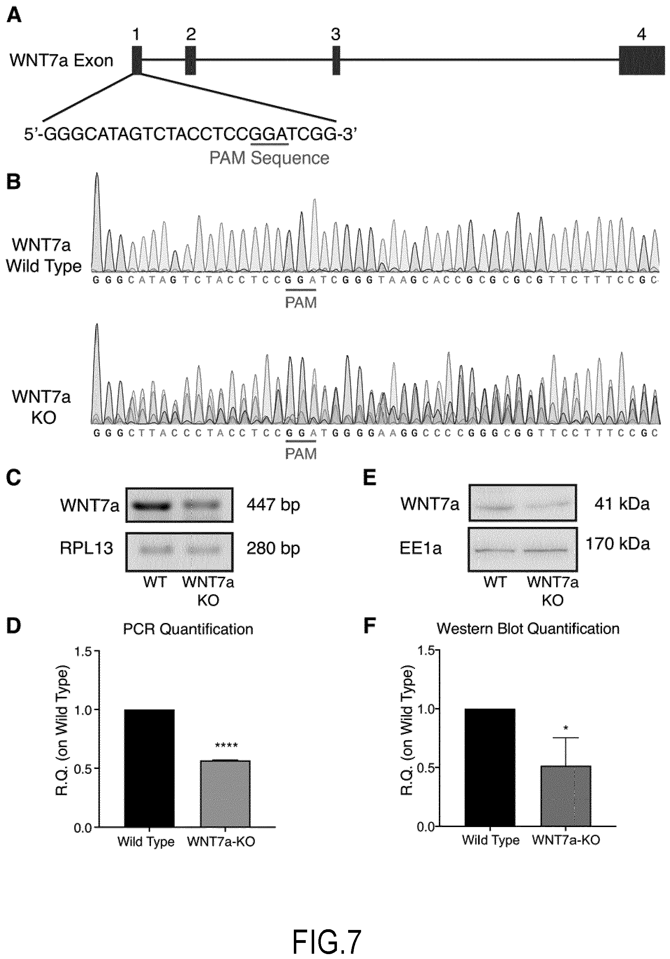

- Figure 7 shows characterization of WNT7a inhibition using CRISPR/Cas9 model in C2C12. Panel A) Graphical representation of the mouse WNT7a-targeted locus. Boxes and lines indicate exons and introns, respectively. The oligo represents sgRNA target sequence and PAM site is marked in red. Panel B) Sanger DNA sequencing were conducted on PCR products amplified from the genomic WNT7a loci of C2C12. Panels C, D) Genomic PCR analysis and quantification of WNT7a inhibition and as housekeeper was used β-actin. Panels E, F) Western Blot analysis and quantification of WNT7a expression in wild type (WT) and WNT7a silenced murine myoblasts (WNT7a-KO). Data information: all data represent mean±SEM of three independent experiments and the results were expressed as fold-change in comparison to wild type cells. The statistical analysis was determined by Student's t-test. *p < 0.05, **p < 0.01.

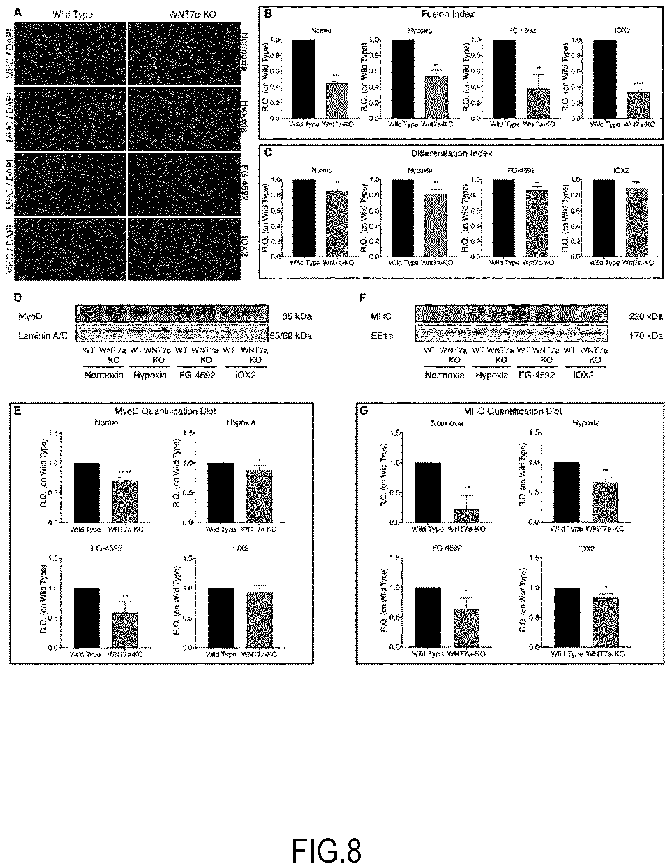

- Figure 8 shows the effects of WNT7a inhibition on skeletal muscle differentiation upon physical and pharmacological hypoxia. Panel A) Immunofluorescence staining of MHC (green) in wild type (WT) and (WNT7a-KO) treated under physical or chemical hypoxia for 24 hours in GM and then switched to DM without the PHDs inhibitors for 7 days. Nuclei were stained with Hoechst 33342. Magnification is 200x. Panel B) Quantification of the fusion index, as the ratio between MHC-positive nuclei and the total number of nuclei. Panel C) Quantification of the differentiation index, as the ratio between myotubes area and MHC-positive nuclei at the end of differentiation. Panels D, E) Western blot analysis and relative quantification of MyoD nuclear localization were tested after 24 h preconditioning under physical or chemical hypoxia in wild type (WT) and WNT7a silenced murine myoblasts (WNT7a-KO). The nuclear marker Lamin A/C was used as the housekeeper. Panels F, G) Western blot analysis and relative quantification of MHC were measured at the end of the skeletal muscle differentiation in wild type (WT) and WNT7a silenced murine myoblasts (WNT7a-KO). The early endosomal antigen 1a (EE1a) was used as the housekeeper. Data information: all data represent mean±SEM of three independent experiments and the results were expressed as fold-change in comparison to wild type cells. The statistical analysis was determined by Student's t-test. *p < 0.05, **p < 0.01, ****p<0.0001.

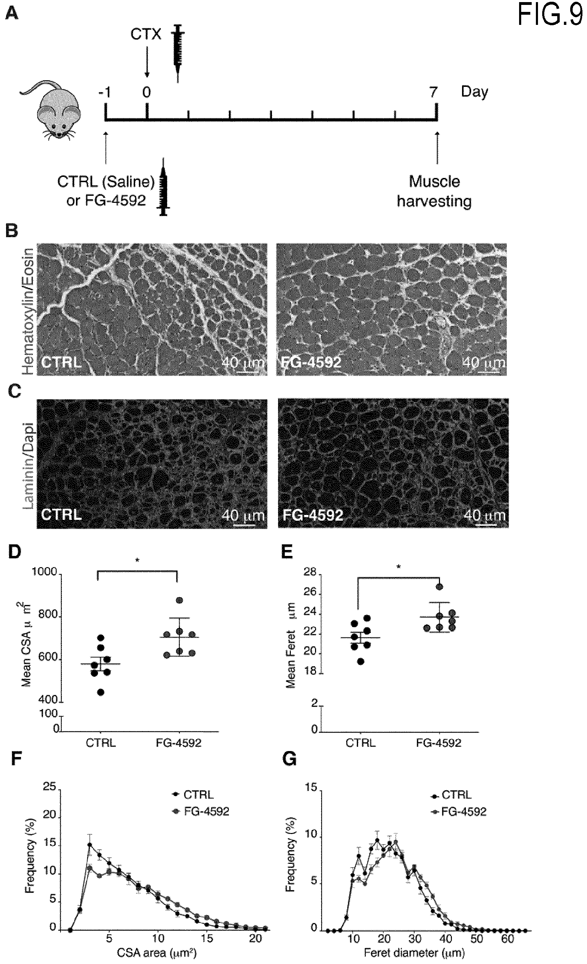

- Figure 9 shows the effects of FG-4592 treatment on muscle regeneration. Panel A) Schematic illustration of the experimental procedure: 24 hours before CTX injury of TA muscle, mice were injected with i.p. of FG-4592 (10mg/kg). Mice were sacrificed 7 days after CTX-induced injury, and TA muscles were collected. Panel B) Immunohistochemical detection of hematoxylin/eosin in FG-4592 pre-treated mice as compared to controls. Magnification is 200x. Panel C) Immunofluorescence staining of laminin (green) in saline and FG-4592 pre-treated mice. Nuclei were stained with Hoechst 33342. Magnification is 200x. Panels D, E) Quantification of CSA and minimal Feret diameter averages in FG-4592 or saline pre-treated mice. Panels F, G) Representation of CSA and minimal Feret diameter distribution in saline or FG-4592 pre-treated mice. Data information: Scale= 40 µM in all panels. The experiment was performed using five and four mice for CTRL and FG-4592, respectively. The statistical analysis was determined by Student's t-test *p < 0.05.

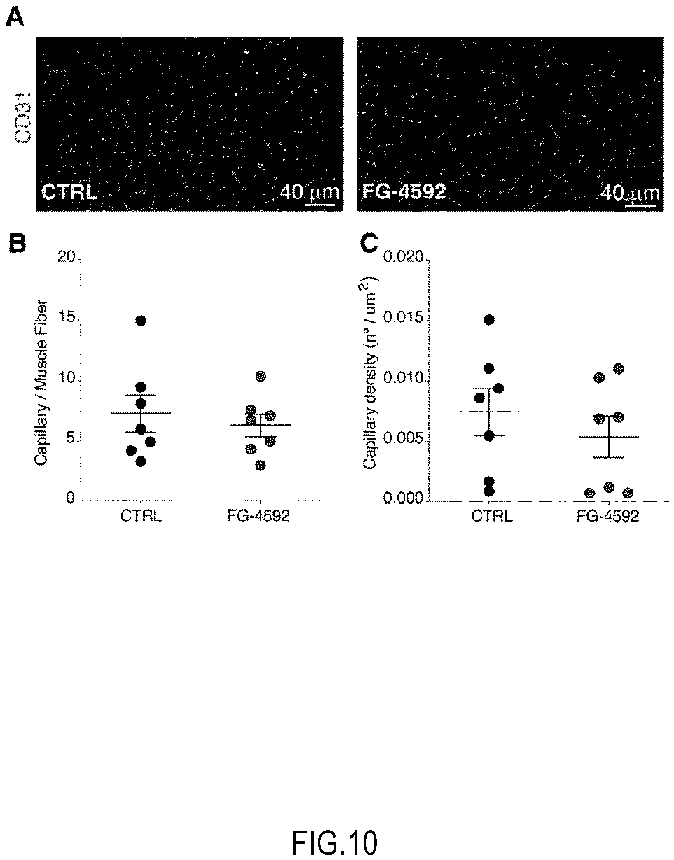

- Figure 10 shows the assessment of angiogenesis in FG-4592 or saline treated mice upon CTX-induced muscle injury. Panel A) Immunofluorescence staining of CD31 (green) on tibial anterior muscle sections of saline and FG-4592 pre-treated 7 days after CTX-induced muscle injury. Magnification is 200x. Panels B, C) Quantification of the total number of capillaries, normalized on the total number of muscle fibers, and the density of capillaries, calculated as the total number of capillaries on the area of the fibers, in saline or FG-4592 pre-treated mice. Data information: Scale= 40 µM in all panels. The experiment was performed using five and four mice for CTRL and FG-4592, respectively. The statistical analysis was determined by Student's *t*-test showing no significant differences (*p > 0.05, n.s.).

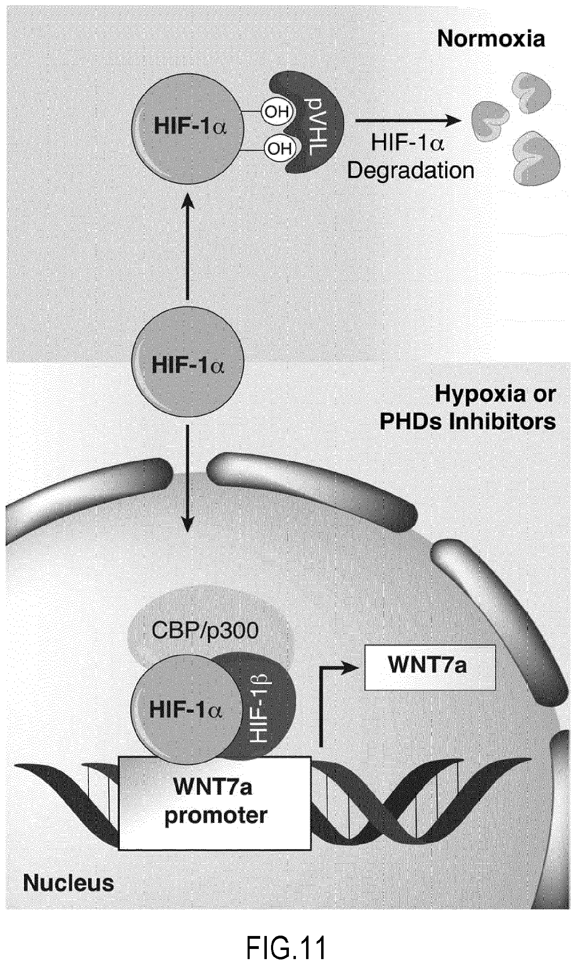

- Figure 11 shows a schematic representation of HIF-1α binding to the WNT7a promoter. Under normoxia, HIF-1α is hydroxylated by the active PHDs and sent to proteasomal degradation. Under hypoxia or upon PHDs inhibition, HIF-1α cannot be hydroxylated and it translocates to the cell nucleus, where it forms a transcriptional complex with its α-subunit and CBP/p300, and binds to specific HRE sequences on the WNT7a promoter region.

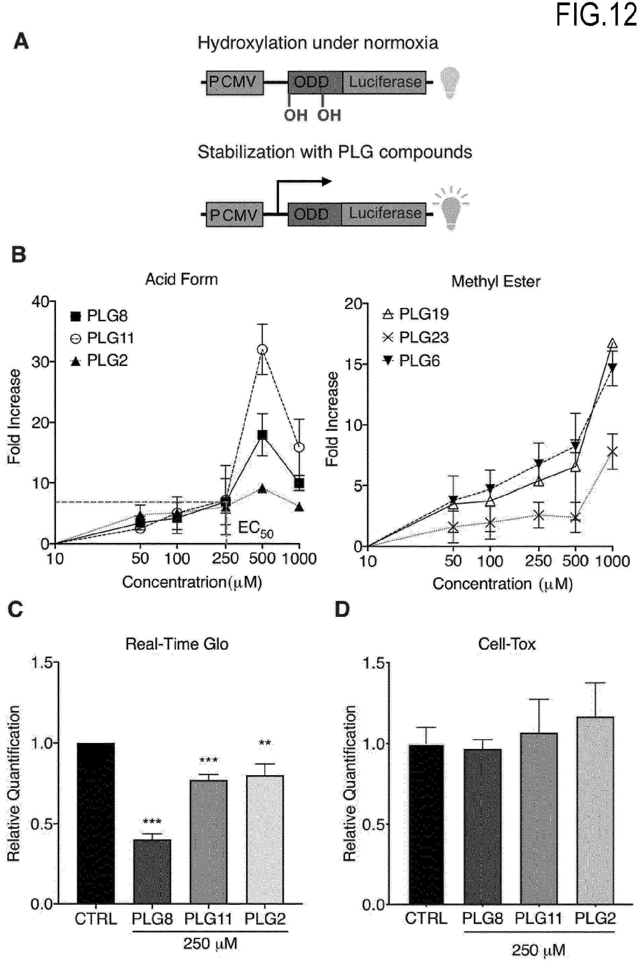

- Figure 12 shows the characterization on PLG compounds in C2C12 murine myoblast and analysis of cell viability. Panel A) Schematic representation of the pcDNA3 construct containing the ODD-luciferase reporter gene. Under normoxia, the proline residues on the ODD sequence are hydroxylated by the PHDs, and the complex degraded by the proteasome, eventually inhibiting the generation of the luminescence signal; the pharmacological inhibition of the PHDs causes the stabilization of the ODD inducing the production of luminescence. Panel B) EC₅₀ quantification of PLG compounds by luciferase assay with the ODD-pcDNA3 construct. Panel C) Cell viability analyzed by with RealTime-Glo™ kits MT Cell Viability Assay upon PLG pre-treatment. Panel D) Cell toxicity measured with CellTox™ Cytotoxicity Assay upon PLG pre-treatment. Data information: all data represent mean ±SEM of three independent experiments and the statistical analysis was determined by Student's *t*-test showing no significant differences **p < 0.01, ***p<0.001.

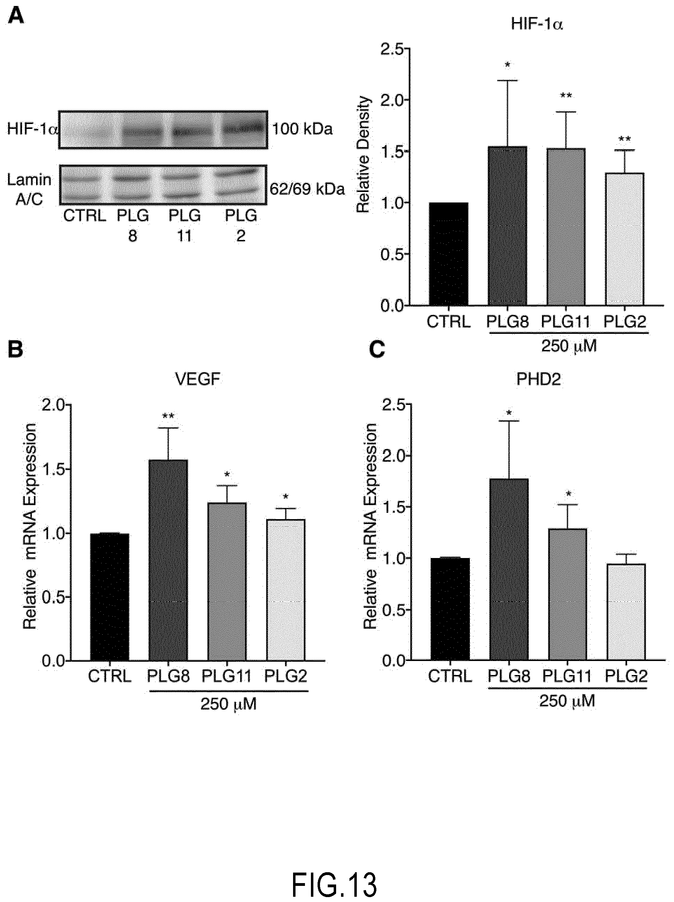

- Figure 13 shows the results of the pharmacological activation of HIF-1α pathway upon PLG pre-treatment. Panel A) Western blot analysis and relative quantification of HIF-1α nuclear localization in murine myoblasts treated with PLG8, PLG11 and PLG2 for 24 hours, the nuclear marker Lamin A/C was used as the housekeeper. Panels B-D) qPCR analysis of HIF-1α target genes, VEGF and PHD2, upon PHDs inhibition with PLG8, PLG11 and PLG2. Data information: all data represent mean±SEM of three independent experiments and the statistical analysis was determined by Student's t-test. *p < 0.05, **p < 0.01.

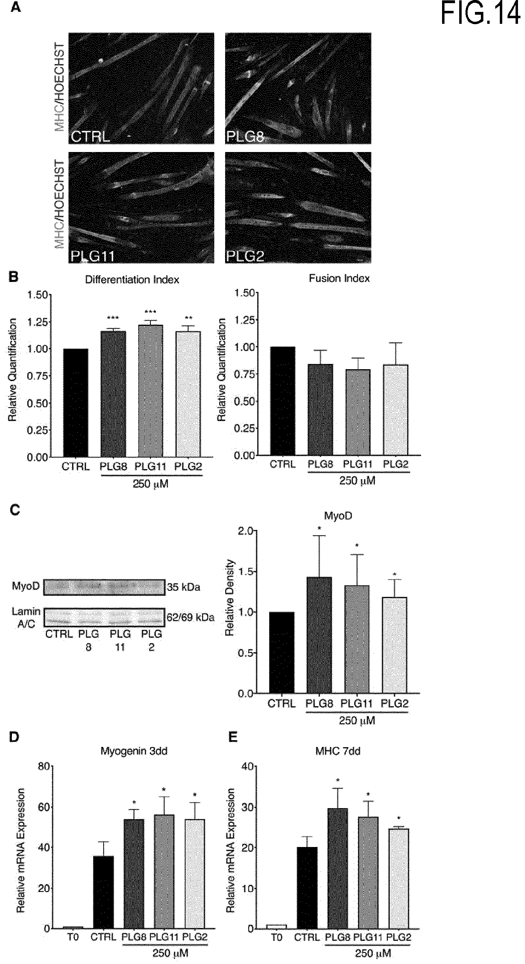

- Figure 14 shows the effects of HIF-1α pharmacological activation on skeletal muscle differentiation. Panel A) Immunofluorescence staining of MHC (green) in murine myoblasts treated with PLG8, PLG11 and PLG2 for 24 hours in GM and then switched to DM without the PHDs inhibitors for 7 days. Nuclei were stained with Hoechst 33342. Magnification is 200x. Panel B) Quantification of the fusion index, as the ratio between MHC-positive nuclei and the total number of nuclei, and of the differentiation index, as the ratio between myotubes area and MHC-positive nuclei at the end of differentiation. Panel C) Western blot analysis and relative quantification of MyoD nuclear localization of murine myoblasts treated for 24 hours with PLG8, PLG11 and PLG2, the nuclear marker Lamin A/C was used as the housekeeper. Panels D, E) qPCR analyses of Myogenin, at three days and of MHC at seven days of differentiation, in murine myoblasts pre-treated with PLG8, PLG11 and PLG2. Data information: all data represent mean±SEM of three independent experiments and the results were expressed as fold-change in comparison to normoxia. The statistical analysis was determined by Student's t-test. *p < 0.05, **p < 0.01, ***p<0.001.

The following examples are merely illustrative and should not be considered limiting the scope of the present invention.

### EXAMPLE 1: Study on the binding of HIF-1α on the WNT7a promoter

### MATERIAL AND METHODS

### Cell culture

C2C12 murine myoblasts (Merck) and HIF-1α-silenced C2C12 (shHIF-1α), which were obtained in our previous study [24], were cultured in growth medium (GM) made of Dulbecco's modified Eagle's medium (DMEM, Merck) with high glucose concentration (4.5 g/L) supplemented with 10% (v/v) fetal bovine serum (FBS, Merck), 2 mM glutamine (Merck), penicillin/streptomycin 1X (Euroclone) (GM) at 37°C in a 5% CO₂ and 95% air-humidified atmosphere. Cells were treated for 24 hours under hypoxic culture conditions (1% O₂) or with PHDs inhibitors (IOX2 or FG-4592) at 37°C in a 5% CO₂ / 21% O₂. In order to induce muscle differentiation, 75 x 10³ C2C12 murine myoblasts were seeded in 35 mm culture plates, cultured in GM for 24 hours, and then switched to a differentiation medium (DM) containing DMEM supplemented with 2% (v/v) horse serum (HS) (Merck) [27]. The DM was changed every day for 7 days. Cells were analyzed at 24 hours post-treatment (PT), at 3^{rd} and 7^{th} days of skeletal muscle differentiation. These cells were tested for mycoplasma contamination before experiments and they are negative.

### WNT7a plasmid construct preparation

Genomic DNA was extracted from C2C12 murine myoblast and used as the template for WNT7a promoter amplification. The PCR reaction was prepared using a Phusion High-Fidelity kit (New England Biolabs) and employing the following primers: forward 5'-GGGGTACCCCGGAAGCTATCACAGGCTT-3' (SEQ ID NO:1), containing KpnI enzymatic site, and reverse 5'-GGGTTCGAACCCTAGCATCCTTCGCTAACT-3' (SEQ ID NO:2) containing the HindIII enzymatic site. The PCR fragment and the vector pNL1.1(Nluc) (Promega) were digested with KnpI and HindIII (Promega) restriction enzymes for 2 hours at 37°C and, then, they were purified from agarose gel using Wizard® SV Gel and PCR Clean-Up System (Promega). The fragment was inserted into the multiple cloning site of pNL1.1 vector using T4 Ligase Fast (Promega) and the reaction was incubated for 1 hour at room temperature. The presence of the insert in the plasmid was finally verified by DNA sequencing.

### Nano-Glo luciferase reporter assay

The regulation of WNT7a promoter mediated by HIF-1α was evaluated with the Nano-Glo® Dual-Luciferase® Assay System kit (Promega), which allows a high-throughput analysis of mammalian cells containing the following reporter genes: (a) Nanoluc (pNL1.1empty and pNL1.1WNT7a_promoter) and (b) pGL4.54(luc2/TK), containing the reporter gene Firefly as internal control. Briefly, cells were seeded in a 96-well plate at a concentration of 5 x 10³ cells per well, transfected with the pNL1.1 or pNL1.1WNT7a_promoter (90 ng) plasmids and pGL4.54(luc2/TK) (10 ng) plasmid as internal control, according to ViaFect Transfection Reagent® protocol (Promega), and then incubated for 24 hours. After transfection, cells were exposed for 24 hours to hypoxic culture conditions (1% O₂) or to the PHDs inhibitors IOX2 (Merck) or FG-4592 (Roxadustat, Selleckchem) at their respective IC₅₀ working concentrations. Then, an equal volume of ONE-Glo™ EX Luciferase Assay Reagent was added to each well, incubated for 30 minutes, and then the luminescence was analyzed with a Varioskan™ LUX Multimode Microplate Reader (ThermoFisher Scientific). To turn off the luminescence of the Firefly luciferase and provide the substrate for the Nanoluc, an equal volume of NanoDLR™ Stop&Glo® Reagent was added to the mixture and incubated for 30 minutes before analysis.

### Chromatin immunoprecipitation (ChIP) assay

The identification of the binding sequence of HIF-1α protein on WNT7a promoter was obtained with a Chromatin Immunoprecipitation Assay (ChIP) using a SimpleChIP® Enzymatic Chromatin IP Kit (Magnetic Beads, Cell Signaling Technology) according to the manufacturer's instructions. Briefly, 1 x 10⁷ C2C12 cells were seeded in GM and cultured for 24 hours under hypoxic (1% O₂) or normoxic (21% O₂) culture conditions. Then, cells were harvested by scraping and the genomic DNA was cross-linked with bound proteins with 1% formaldehyde for 10 minutes at room temperature. Then, the genomic DNA was digested with Microccocal Nuclease for 20 minutes at 37°C to achieve DNA sizes ranging from 150 to 900 bp, and then subjected to sonication (Branson Sonifier 150). The immunoprecipitation was achieved using: (a) rabbit monoclonal anti-HIF-1α, 1:1000 dilution (clone D2U3T, Cell Signaling Technologies), (b) rabbit monoclonal anti-Histone-H3 as technical positive control, 1:50 dilution (D2B12, Cell Signaling Technologies) and (c) IgG rabbit, 5 µg, as the negative control. The mixture was then incubated at 4°C over-night under rotation. The immuno-complexes were collected with ChIP Grade Protein G Magnetic Beads and incubated for 2 hours at 4°C under rotation. The elutes were digested with Proteinase K at 65°C for 4 hours, and the precipitated DNA was recovered using DNA Purification Spin Columns. Finally, the purified DNA was amplified by Real-Time PCR using the following ChIP primers designed on the WNT7a promoter: WNT7al forward 5'-GGAAGCTATCACAGGCT-3' (SEQ ID NO:3), and reverse 5'-ACCGTGGATTCAAGGA-3' (SEQ ID NO:4); WNT7a2 forward 5'- TGTCAGCATGGCTGGCGC-3'(SEQ ID NO:5) and reverse 5'-AGTTCTTCATGCATAC-3' (SEQ ID NO:6); WNT7a3 forward 5'-GCAGTCTGGAAGGCTAAGC-3' (SEQ ID NO:7) and reverse 5'-GTCAAACTCAAAGTCAC-3' (SEQ ID NO:8); WNT7a4 forward 5'-TTCACCTCCTCAGTTTCTCCA-3'(SEQ ID NO:9) and reverse 5'-AGCCCAATGACCAGTAGCAG-3' (SEQ ID NO:10); WNT7a5 forward 5'-TCCAGTCCAATTCCAAGTC-3' (SEQ ID NO:11) and reverse 5'-GTCTGTGCGAGGGGCTGA-3' (SEQ ID NO:12); WNT7a6 forward 5'-GAGGGTGAGGAGAGAATG-3' (SEQ ID NO:13) and reverse 5'-CCTTGTTAGAGCTCTGTCA-3' (SEQ ID NO:14); WNT7a7 forward 5'-CGTGAGGGTTCTGAAATG-3'(SEQ ID NO:15) and reverse 5'-GGAATCCTGCCTGCCTAG-3'(SEQ ID NO:16).

### Site-directed mutagenesis

The identification of HIF-1α binding sites on WNT7a promoter was performed by site-directed mutagenesis experiments followed by luciferase assays. Each HRE sequence, which was identified as a putative binding site for HIF-1α on the WNT7a promoter, was deleted using the QuickChange Lightning Site-Directed Mutagenesis kit (Agilent). Briefly, in order to generate a mutant plasmid containing a single HRE deletion, a thermal cycling reaction mix was performed using pNL1.1WNT7a_promoter as the template, together with two synthetic mutagenic primers suitably designed to insert the desired deletion. Following the temperature cycling, the product was treated with Dnp I endonuclease, which recognized both methylated and hemimethylated DNA, to digest the parental DNA template and to select the mutant DNA. The vector DNA containing the deletion was then transformed into XL10-Gold ultracompetent cells. Each mutant plasmid was subjected to DNA sequencing to confirm the correct deletion.

### RESULTS

### HIF-1α binding on the WNT7a promoter

To test whether HIF-1α activation was directly responsible for WNT7a transcriptional induction, HIF-1α-silenced (kd-HIF-1α) and controls (HIF-1α) murine myoblasts were seeded to set up a luciferase assay. They were transiently co-transfected with plasmid pNL1.1WNT7a_promoter, containing the luciferase gene under the control of the WNT7a promoter (Figure 1, panel A), and with plasmid pGL4.54 (luc2/TK), containing the luciferase gene under the TK promoter activity as the internal control. After transfection, cells were switched to hypoxic culture conditions (1% O₂) for 24 hours and, then, tested for WNT7a promoter activation by luciferase assay. Results revealed a 2.5-fold luminescence increase in WT myoblasts upon the hypoxic treatment, as compared to normoxic controls, which was consistent with an enhancement of the WNT7a promoter activity under hypoxia (Figure 1, panel B).

On the other hand, a 40% silencing of HIF-1α nuclear translocation, tested by WB, (Figure 2, panels A, B) caused a significant reduction (-40% luminescence) in WNT7a promoter activation under hypoxia, as compared to wild type myoblasts (Figure 2, panels C, D). Next, quantitative ChIP (qChIP) assays were performed to identify any binding regions of HIF-1α on the WNT7a promoter by dividing it into seven putative binding sequences from -1000 bp to -197 bp, each containing at least one Hypoxia Responsive Element (HRE) (5'-CGTG-3') (Figure 1C). After qChIP with the HIF-1α antibody, each sequence was amplified by qPCR with specific primers, revealing no significant binding under normoxia. Contrarily, results showed that, under hypoxia, HIF-1α bound to Seq.1 (-1000 - 843 bp) and Seq.4 (-653 -489 bp) (Figures 1, panel and Figure 3), each containing one and three HRE sequences, respectively (Figure 1, panel E). To further discriminate among four HRE sites, which was responsible for HIF-1α binding, site-directed mutagenesis experiments were performed to generate four mutants of the WNT7a promoter (1.1, 4.1, 4.2 and 4.3), each carrying a single HRE deletion (Figure 1, panel E). Murine myoblast C2C12 were transiently co-transfected with plasmid pNL1.1-WNT7a (Wild Type or one of the mutants) and with plasmid pGL4.54(luc2/TK), as the internal control, and then cultured under hypoxic conditions (1% O₂) for 24 hours. Luciferase assays revealed a 40% decrease in luminescence for deletion 1.1 and 4.2, while no significant differences could be observed for mutants 4.1 and 4.3, as compared to WT, supporting that only HREs 1.1 and 4.2, localized on sequence 1 and 4, respectively, were responsible for HIF-1α binding on the WNT7a promoter (Figure 1, panel F).

The study allows to discover that HIF-1α can regulate WNT7a gene expression by directly binding to its promoter as schematically represented in Figure 11. Figure 11 illustrates that under normoxia, HIF-1α is hydroxylated by the active PHDs and sent to proteasomal degradation. Under hypoxia or upon PHDs inhibition, HIF-1α cannot be hydroxylated and it translocates to the cell nucleus, where it forms a transcriptional complex with its α-subunit and CBP/p300, and binds to specific HRE sequences on the WNT7a promoter region.

### EXAMPLE 2: In vitro study on the effect of PHDs inhibitors IOX2 and FG-4592

### MATERIAL AND METHODS

### Determination of the IC₅₀ for the PHDs inhibitors IOX2 and FG-4592

The IC₅₀ was calculated using a Dual-Glo® Luciferase Assay System kit (Promega) that allows a high-throughput analysis of mammalian cells containing the Firefly (Luciferase-pcDNA3, ODD-Luciferase-pcDNA3) and Renilla (pRL-CMV) luciferase reporters. Briefly, 5 x 10³ cells per well were seeded in a 96-well plate, and then transfected for 24 hours with a Luciferase-pcDNA3 or an ODD-Luciferase-pcDNA3 (80 ng), containing the firefly gene reporter. A co-transfection with pRL-CMV (8 ng), containing the renilla gene reporter, was performed as an internal control, according to the ViaFect Transfection Reagent® protocol (Promega). After transfection, cells were treated with 10, 25, 50, and 100 µM IOX2 or FG-4592 (R oxadustat) for 24 hours, and then an equal volume of Dual-Glo® Luciferase Reagent was added to each well, incubated at room temperature for 30 minutes, and then analyzed with a Varioskan™ LUX Multimode Microplate Reader (ThermoFisher Scientific). Then, an equal volume of Dual-Glo® Stop & Glo® Reagent was added to each well in order to quench the luminescence from the firefly reaction and to provide the substrate for the renilla luciferase. The mixture was incubated for 30 minutes before analysis. The IC₅₀ values were calculated by linear regression.

### Cell viability assay

RealTime-Glo™ kits MT Cell Viability Assay (Promega) was used to investigate the effects of IOX2 and FG-4592 treatment on cell viability. Briefly, 5 x 10³ C2C12 murine myoblasts were seeded in a 96 well plate and incubated for 24 hours (T0) with MT Cell Viability Substrate (1:1000 dilution) and NanoLuc® Enzyme (1:1000 dilution). Then, 50 µM IOX2 or 40 µM FG-4592 were added to the cell cultures and incubated for other 24 hours before luminescence analysis.

### Cell toxicity assay

To assess PHDs inhibitors cytotoxicity, 5 x 10³ C2C12 murine myoblasts were seeded in a 96-well plate and treated with 50 µM IOX2 or 40 µM FG-4592 for 24 hours before analysis with a CellTox™ Cytotoxicity Assay (Promega). Briefly, 100 µl of CellTox Buffer containing CellTox Green Dye (1:500 dilution) was added to each well and incubated at room temperature for 15 minutes before fluorescence analysis (excitation wavelength of 485-500 nm and emission filter of 520-530 nm).

### RNA extraction and gene expression by Real-Time Quantitative PCR (qPCR)

Total RNA was isolated using ReliaPrep™ RNA Miniprep Systems (Promega), and reverse transcribed to cDNA using the iScript cDNA synthesis kit (BioRad), according to the Manufacturer's instructions. Real-Time Quantitative PCR was performed on 10 ng of cDNA template, 0.2 µM primers, and GoTaq® qPCR Master Mix (Promega) in 20 µl final volume using a StepOnePlus® Real-Time PCR System (Applied Biosystem). The following primers were used: VEGF forward 5'-AAAAACGAAAGCGCA-3' (SEQ ID NO:17) and reverse 5'-TTTCTCCGCTCTGAA-3' (SEQ ID NO:18); PHD2 forward 5'-CTGTGGAACAGCCCTTTTT-3' (SEQ ID NO:19) and reverse 5'-CGAGTCTCTCTGCGAATCCT-3' (SEQ ID NO:20); MYOG forward 5'-AGCCACACTGAGGGA-3' (SEQ ID NO:21) and reverse 5'-GTTGAGGGAGCTGAG-3' (SEQ ID NO:22); MHC forward 5'-TGGAGCAGGAGGAATACAAG-3' (SEQ ID NO:23) and reverse 5'-GCATAGTGGATGAGGGAGAA-3' (SEQ ID NO:24); WNT7a forward 5'-ACTGTGGCTGCGACAAG-3' (SEQ ID NO:25) and reverse 5'-CTTCATGTTCTCCTCCAG-3' (SEQ ID NO:26); MYOR forward 5'-GCCCAGCGACATTTCTTC-3' (SEQ ID NO:27) and reverse 5'-CGCTTCCTCTTGCATCCT-3' (SEQ ID NO:28); RPL13 forward 5'-CTCGGCCGTTCCTGTAT-3' (SEQ ID NO:29) and reverse 5'-GTGGAAGTGGGGCTTCAGTA-3' (SEQ ID NO:30). RPL13 was used as the housekeeping gene. The amplification program consisted in an initial denaturation at 95°C for 3 minutes, followed by 40 cycles of 5 seconds each at 95°C and 30 seconds at 57°C; WNT7a was amplified at 53°C. Relative quantification of target genes was performed in triplicate and calculated by the equation 2^{-ΔΔCt}.

### Total proteins extraction and isolation of the nucleus compartment

For total proteins extraction, 7,5 x 10⁴ cells were seeded and harvested at 7^{th} days of skeletal muscle differentiation, as previously described. Cells were collected after enzymatic digestion with trypsin (Merck), lysed by sonication and centrifuged at 800 xg for 10 minutes. Supernatant was used to determine total protein content with the BCA Protein Assay Kit (Pierce), according to the manufacturer's instructions.

For nuclei separation, 5 x 10⁶ cells were collected after enzymatic digestion with trypsin (Merck), centrifuged at the maximum speed, resuspended in 400 µl of Buffer A (10 mM KCl, 0.1 mM EDTA, 1 mM DTT, 10 mM Hepes, pH 7.9), containing protease and phosphatase inhibitors cocktail (Merck), and incubated on ice for 20 minutes. Then, 10% NP40 was added to the cell suspension, mixed for 40 seconds, and centrifuged 40 seconds at the maximum speed. After centrifugation, the pellet was resuspended in 80 µl of Buffer C (0.4M NaCl, 1 mM EDTA, 1 mM DTT, 20 mM Hepes, pH 7.9), containing a protease and phosphatase inhibitors cocktail, and then mixed for 30 minutes at 4°C at the maximum speed. Finally, the nuclear suspension was centrifuged for 5 minutes at 4°C at the maximum speed, collected and transferred in a new tube. The total protein content was determined with the BCA Protein Assay Kit (Pierce), according to the manufacturer's instructions.

### Western blot analyses

Proteins were denaturated by boiling for 10 minutes in sample buffer (0.6 g/100 mL Tris, 2 g/100 mL SDS, 10% glycerol, 1% 2-mercaptoethanol, pH 6.8) and loaded into 10% SDS-PAGE gel, then transferred onto a nitrocellulose membrane (Trans-blot, Bio-Rad Laboratories) by electroblotting. Nitrocellulose membranes were incubated with a blocking solution containing 5% (w/v) non-fat dry milk or 5% (w/v) BSA (Merck) in Tris-buffer saline with 0.1% Tween® 20 (TBS-T) for 1 hour. Blots were incubated for 2 hours at room temperature with the following primary antibodies: rabbit monoclonal anti-HIF-la, 1:1000 dilution (clone D2U3T, Cell Signaling Technology), goat polyclonal anti-Lamin A/C, 1:1000 dilution (clone N-18, Santa Cruz Biotechnology), mouse monoclonal anti-MyoD1, 1:1000 dilution (clone 5.2F, Abcam), mouse monoclonal anti-myosin (skeletal, fast), 1:1000 dilution (clone MY-32, Merck) and rabbit polyclonal anti-EE1a, 1:1000 dilution (Cell Signaling Technology). Membranes were washed three times for 10 minutes with TBS-T, and then incubated with the appropriate anti-mouse, anti-rabbit, or anti-goat HRP-conjugated secondary antibodies (Dako, Agilent Technologies), 1:2000 dilution, for 1 hour at room temperature. After three washes for 10 minutes with TBS-T, the immunoreactive bands were visualized using the enhanced chemiluminescence detection kit reagents (ECL Advance, GeHealthcare), according to the manufacturer's instructions.

### Immunofluorescence staining

After differentiation for 7 days in DM, cells were washed with phosphate saline buffer (PBS) and fixed for 15 minutes in 4% (w/v) paraformaldehyde at room temperature. For permeabilization and blocking, cells were incubated for 1 hour in the presence of PBS 0.1% (v/v) Triton X-100 (TX-100, Merck) and 5% (w/v) FBS (Merck) at room temperature. Then, cells were incubated for 2 hours at room temperature with mouse monoclonal anti-Myosin (Skeletal, fast) (clone MY-32, Merck), diluted 1:200 in PBS 0.1% (v/v) Triton X-100 (TX-100) and 5% (w/v) FBS. After incubation, cells were washed three times in PBS and incubated for 1 hour at room temperature with an anti-mouse FITC-conjugated secondary antibody (Jackson ImmunoResearch), diluted 1:200. Cell nuclei were counterstained with Hoechst 33342 (1:500 dilution, Merck). Myogenesis was assessed by measuring the myotube area and the number of myonuclei per myotube using a fluorescent microscope (Olympus TH4-200) equipped with an acquisition camera. To quantify both differentiation and fusion indexes, ten fields were chosen randomly and, for each field, a minimum of one hundred myosin-positive myotubes with more than two myonuclei were measured using the ImageJ v1.49o software. The area and the number of nuclei per myotube was the mean of ten measurements averaged from three different experiments.

### RESULTS

### The pharmacological activation of HIF-1α induces WNT7a transcription

Since HIF-1α can directly activate WNT7a transcription, it was assessed if the stabilization of HIF-1α could be also obtained using two PHDs inhibitors, IOX2 and FG-4592 (Maxwell and Eckardt, 2016). To this purpose, the IC₅₀ of both compounds was determined by transiently co-transfecting C2C12 myoblasts with the ODD-luciferase-pcDNA3 plasmid, containing the Oxygen Dependent Domain (ODD) of HIF-1α, and with the pRL-CMV plasmid, as the internal control (Figure 4, panel A). Then, transfected cells were treated for 24 hours with IOX2 or FG-4592 at different concentrations (10, 25, 50, and 100 µM) under normoxic conditions, and the emitted luminescence was collated to untreated controls. Results showed an IC₅₀ of 50 µM and 40 µM for IOX2 or FG-4592, respectively (Figure 4, panel B). Cell viability and toxicity analyses at IC₅₀ concentrations showed no significant changes as compared to untreated controls (Figure 5, panels A, B). Furthermore, the activation of HIF-1α and its main target genes were evaluated. Unlike the untreated control, both IOX2 and FG-4592 treatments induced HIF-1α nuclear translocation, with an 8.8- and 15-fold protein increase, respectively (Figure 4, panel C). Moreover, qPCR analyses of HIF-1α target genes demonstrated that IOX2 treatment induced a 1.6- and 4.4-fold increase in the expression of VEGF and PHD2, respectively, while FG-4592 promoted a 2.1- and 4.8-fold increase of the same genes, as compared to untreated controls (Figure 4, panel D). Successively, the activation of the WNT7a promoter was investigated by luciferase assay, revealing that IOX2 and FG-4592 treatments induced a 1.4- and 1.3-fold luminescence increase, respectively (Figure 4, panel E). Finally, qPCR experiments revealed a 1.4- and 2.3-fold increase in WNT7a gene expression (Figure 4, panel F).

### Treatment with PHDs inhibitors induces myogenesis

To assess whether a pharmacological activation of HIF-1α would affect myogenesis, murine myoblasts C2C12 were pre-treated with IOX2 or FG-4592 in GM for 24 hours, and then induced to differentiate for seven days by switching them to DM without the PHDs inhibitors. At the end of the differentiation process, an extensive formation of myosin heavy chain (MHC) positive myotubes was sighted in both pre-treated and untreated cells (Figure 6, panel A). Quantitative evaluation of the differentiation parameters revealed no significant changes in the fusion indexes, whereas a 1.5- and 1.3-fold increase in the differentiation indexes could be observed in IOX2 and FG-4592 pre-treated myoblasts, respectively, as compared to untreated controls (Figure 6, panel B). Protein expression analyses revealed that IOX2 and FG-4592 induced a 1.6- and 1.5-fold increase, respectively, of the nuclear localization of the early differentiation marker, MyoD (Figure 6, panel C). Along this line, the mRNA level of the main MyoD corepressor, MYOR, was found down-regulated of a 2.2- and 2.6-fold, as compared to the controls (Figure 6, panel D).

Finally, gene expression analyses showed a 2.0- and 1.4-fold increase of Myogenin at day 3, and a 2.2- and 2.3-fold enhancement of MHC at day 7 of differentiation in IOX2 and FG-4592 pre-treated myoblasts, respectively (Figure 6, panels E, F).

### EXAMPLE 3: Study of the effects on myogenesis by conducing WNT7A silencing by Crispr/Cas9 genome editing in C2C12 murine myoblasts

The results of Example 2 supported the hypothesis that a physical or chemical activation of HIF-1α is directly involved in the induction of WNT7a gene expression leading to the formation of hypertrophic myofibers.

To further validate this theory, the inventor tested if silencing of WNT7a reduced skeletal muscle hypertrophy upon HIF-1α activation.

Therefore, WNT7a silencing was conducted by Crispr/Cas9 genome editing in C2C12 murine myoblasts and the effects on myogenesis were evaluated. To this purpose, Wild Type and WNT7a-KO murine myoblasts were cultured for 24h in normoxia, or in physical (1% O₂) or chemical hypoxia (IOX2/FG-4592) and then cells were induced to differentiate under normoxic conditions for 7 days. Data showed that the silencing of WNT7a strongly reduced the differentiation process, as compared to Wild Type, inducing a marked decrease of MyoD nuclear translocation and MHC protein expression, ultimately leading to a reduction of fusion and differentiation indexes.

### CRISPR/Cas9 mediated knockout of WNT7a gene in murine myoblast C2C12

The knockout of WNT7a gene was obtained performing CRISPR/Cas9 genome editing. In particular, crRNAs and tracrRNA were obtained from TrueGuide™ Synthetic gRNA kit (ThermoFisher Scientific) and they were reconstituted and annealed following the manufacturer's instructions. In particular, the target sequence 5'-GGGCATAGTCTACCTCCGGATCGG-3' (SEQ ID NO:31) was selected as the crRNA of the *WNT7A* gene, while the target sequence 5'-AAAUGUGAGAUCAGAGUAAU-3' (SEQ ID NO:32), which doesn't recognize any sequence in the human genome (ThermoFisher Scientific), was used as the negative control. Briefly, they were re-suspended using 1X Tris-EDTA buffer pH 8.0 to prepare a 100 µM stock solution. Then, gRNAs were generated preparing a mix composed of 10 µL crRNA, 10 µL tracrRNA, 10 µL annealing buffer, and 20 µL nuclease-free water. The mixture was incubated at 95 °C for 5 minutes followed by 10 mins on 78 °C, and then 25 °C for 5 minutes.

The transfection was performed seeding 5 x 10⁵ murine myoblasts C2C12 in a 24-well plate. Two different mixtures were prepared:
- 1250 ng of TrueCut™ Cas9 Protein v2 (ThermoFisher Scientific), 2.5 µl of Lipofectamine™ Cas9 Plus™ Reagent, 240 ng of gRNA and 25 µl of Opti-MEM I Medium
- 1.5 µl Lipofectamine ™ CRISPRMAX ™ reagent (ThermoFisher Scientific) and 25 µl of Opti-MEM I Medium

The diluted Lipofectamine ™ CRISPRMAX ™ reagent in Opti-MEM I Medium was incubated 1 minute at room temperature and, then, it was added to gRNA/Opti-MEM I solution for 15 minutes. Then, the mixture was added to murine myoblasts and it was incubated at 37°C for 2 days. After the incubation, single-cell clones were isolated using a limiting dilution cloning in 96-well plates. The efficiency of WNT7a knockout (WNT7a-KO) was verified by sequencing.

### Genomic DNA extraction and amplification by PCR

Genomic DNA was isolated using Wizard® Genomic DNA Purification kit (Promega), according to the Manufacturer's instructions, and it was used to amplify WNT7a and RPL13 genes using the following primers: WNT7a forward 5'-CTTGTTGCGCTTGTTCTCC-3' (SEQ ID NO:33) and reverse 5'-CGCAATTCCACAGACTCG-3' (SEQ ID NO:34); RPLI 5'-CTCGGCCGTTCCTGTAT-3' (SEQ ID NO:35) and reverse 5'-GTGGAAGTGGGGCTTCAGTA-3' (SEQ ID NO:36). The amplification program consisted in an initial denaturation at 98°C for 30 seconds, followed by 30 cycles of 10 seconds each at 98°C, 15 seconds at 57°C and 30 seconds at 72°C. The amplification was concluded by a final extension step at 72°C for 10 minutes.

### RESULTS

### WNT7a silencing impairs skeletal muscle hypertrophy mediated by HIF-1α activity

To further understand if the effects caused by HIF-1α on skeletal muscle hypertrophy were directly mediated by WNT7a activity, the silencing of WNT7a was performed using the CRISPR/Cas9 genome editing in murine myoblasts C2C12. WNT7a gene is characterized by 4 exons and a gRNA sequence targeted into exon 1 was selected (Figure 7, panel A). As a negative control was employed a non-targeting gRNA sequence, that doesn't recognize any sequence in the human genome. PCR products of the WNT7a targeted genomic region were analyzed by Sanger sequencing confirming the introduction of a frameshift in the gene (Figure 7, panel B). To quantify WNT7a silencing, quantitative PCR and Western Blot analysis were performed on wild-type and WNT7a-KO myoblasts. In particular, results revealed that WNT7a-KO cells showed a 44% decrease in WNT7a genomic expression (Figure 7, panels C, D) and a 49% reduction of WNT7a total protein (Figures 7, panels E, F), as compared to wild-type cells. Then, wild-type and WNT7a-KO cells were cultured for 24 hours in physical or chemical hypoxia and, then, induced to differentiate for 7 days. At the end of the differentiation process, cells were stained for MHC (Figure 8, panel A). The effects of WNT7a silencing have been already seen in normoxia conditions, indeed, it was detected a decrease of 56% and 15% in fusion and differentiation indexes, respectively (Figure 8, panels B, C, Normoxia Line). The same results were also found upon HIF-1α activation. In particular, WNT7a-KO cells demonstrated a 46% and 20% reduction in fusion and differentiation indexes under physical hypoxia (Figure 8, panels B, C, Hypoxia Line). Along this line, chemical pre-treatment perfectly mimicked the effects observed upon physical hypoxia. Indeed, WNT7a-KO cells showed a 63 % and 67% decrease in fusion index upon FG-4592 and IOX2 administration (Figure 8, panel B, IOX2 and FG-4592 Line). Finally, it was observed a 15% reduction in differentiation index induced by FG-4592 treatment, while no significant changes were observed in IOX2 pre-treated myoblasts as compared to wild type cells (Figure 8, panel C, IOX2 and FG-4592 Line). To further confirm the inhibition of myogenesis induced by WNT7a upon HIF-1α activation, MyoD and MHC protein expression analysis were conducted, unveiling that the silencing of WNT7a caused a marked reduction of MyoD nuclear localization. In particular, WNT7a-KO cells showed a decrease of 29%, 13%, 42% in normoxia, hypoxia and upon FG-4592 pre-treatment, respectively, while no significant changes were induced by IOX2 administration, as compared to wild type cells (Figure 8, panels D, E). Along this line, WNT7A-KO cells also demonstrated a marked decrement of MHC expression at the end of the differentiation process. Indeed, MHC decreased by 80%, 34%, 36% and 18% in normoxic, hypoxic, FG-4592 and IOX2 pre-treated myoblasts, respectively (Figure 8, panels F, G).

### EXAMPLE 4: Effects of FG-4592 injection on skeletal muscle regeneration in vivo

### MATERIALS AND METHODS

### Animals

The procedure involving mice was performed according to the animal protocol guidelines described by the Institutional Animal Care and Use Committee (IACUC) authorization no. 89-2018-PR at San Raffaele Scientific Institute (Milan, Italy). All mice were housed for two weeks in individual cages with 12 hours light/dark cycle, allowing free access to food and water. All efforts were made to minimize animal suffering and to reduce the number of mice used, in accordance with the European Communities Council Directive of November 24, 1986 (86/609/EEC). The numbers of mice estimated sufficient to detect a difference between two means as large as 1 SD unit with 80% power and a significance level of 95% at Student's t test were calculated with the program by R.V. Lenth (www.stat.uiowa.edu/~rlenth/Power/index.html) and no formal randomization procedure was used. The investigators conducting the experiments were blind to the experimental group assessed. The investigators quantifying the experimental outcomes were maintained blinded to the animal group or intervention. Finally, the statistic evaluation of the experimental data was performed by another investigator not directly involved in data collection and parameter measurement.

### Cardiotoxin-induced muscle regeneration and exogenous FG-4592 administration

Experiments on muscle regeneration were conducted on 8-10 weeks old male C57Bl/6N mice, matched for weight, and purchased from Charles River Laboratories (Calco, Italy).

Cardiotoxin (CTX, from Naja mossambica, Latoxan, Portes-les-Valence, France, http://www.latoxan.net/) was dissolved in sterile saline to a final concentration of 10 µM. Mice were anesthetized by isofluorane inhalation and hindlimbs were shaved and cleaned with alcohol. Tibialis anterior (TA) muscles were injected with 45 µl of CTX with a 30-gauge needle, with 15 micro-injections of 3 µl CTX each in the mid-belly of the muscle to induce a homogeneous damage. The TA muscles of the contralateral hindlimbs were injected with a saline solution. A 50 mg/ml of FG-4592 stock solution was firstly prepared in DMSO, then further diluted in sterile PBS to 1 mg/ml and stored aliquoted at -20°C.

FG-4592 was administrated 24 hours before cardiotoxin injury by i.p. injection via 31-gauge needles as a typical dose of 10 mg/kg, as already described in literature [25]. DMSO, diluted at 2% in saline solution, was inoculated by i.p. injection to control mice. The experiments were conducted using five mice for each group, although one mouse of the FG-4592 group died spontaneously during the procedure. Mice were sacrificed seven days after cardiotoxin treatment.

### Histological analysis

Histological analysis was performed as we previously reported [26]. Briefly, TA muscles were frozen in liquid nitrogen-cooled isopentane and mounted in Killik embedding medium (Bio-optica). Transverse muscle sections (7 µm) were cryosectioned from the midbelly of each muscle. Sections were stained with Hematoxylin (Bio-optica)/Eosin (Merck) to reveal general muscle architecture. For immunofluorescence, after fixing in PFA 4% for 10 minutes, slices were permeabilized with 0.2% Triton X-100 in 1% BSA for 15 minutes and blocked with 4% BSA for 1 hour. The following primary antibodies anti-laminin (1:200; Dako, Agilent Technologies), anti-CD31 (1:100; Space srl) were incubated overnight at 4°C, while the incubation with the secondary antibody (1:450, anti-rabbit, Alexa Fluor™ Antibodies) was performed at room temperature for 1 hour. Finally, the slices were incubated with Hoechst 33342 (Merck) for 15 minutes. Images were acquired using Axio Lab.A1 (Zeiss) and quantified with ImageJ v1.49o software.

### Statistical analysis

The Student's *t*-test was used to determine the significance values. P values of less than 0.05 were considered to be significant. All P values were calculated from data obtained from at least three independent experiments. Statistical significance was assumed for *p < 0.05. All error bars represent the standard deviation of the mean.

### RESULTS

### Effects of FG-4592 injection on skeletal muscle regeneration in vivo

To assess whether a pharmacological activation of HIF-1α would affect skeletal muscle regeneration, C57Bl/6N mice were pre-treated with a single intraperitoneal injection of FG-4592 (10 mg/kg) or saline solutions, one day before CTX-induced degeneration of the anterior tibialis (AT) muscles (Figure 9, panel A). Mice were sacrificed seven days after injury and, while Hematoxylin/Eosin analyses revealed no macroscopic difference between FG-4592 injected and control muscles (Figure 9, panel B), laminin staining pointed out a 25% enhancement of cross-sectional area (CSA) and an 8% increase of minimal Feret diameter of regenerating myofibers, characterized by centrally located nuclei, in FG-4592 pre-treated mice (Figure 9, panels C, D, E). These results were also confirmed by CSA and minimal Feret diameter distribution analyses, proving that FG-4592 injection induced a shift of a frequency distribution toward bigger fibers (Figure 9 panels F, G). Finally, to exclude if the effects on muscle regeneration could be due to the activation of angiogenesis induced by FG-4592, a CD31 staining was performed on the muscle sections (Figure 10, panel A). Results indicated that FG-4592 treatment did not significantly modify the number per regenerating fibers nor the density of capillaries at seven days from the muscle injury, as compared to control mice (Figure 10, panels B, C) supporting that FG-4592 promoted muscle regeneration independently from angiogenesis activation.

### EXAMPLE 5: Study on the activity of the effect of the diacyl-hydrazine compounds of the invention on HIF

The results of Example 2 supported the hypothesis that a chemical activation of HIF-1α is directly involved in the formation of hypertrophic myotubes. To further confirm this theory, the inventor decided to test the following substituted diacyl-hydrazine (PLG2, PLG6 and PLG19) and the corresponding methyl esters (PLG11, PLG8 and PLG23), already described in the literature for other uses [28]. The effects on skeletal muscle differentiation were analyzed *in vitro.* To this purpose, murine myoblasts were cultured for 24h with the PLG inhibitors and then cells were induced to differentiate under normoxic conditions for 7 days. Data showed that the pre-treatment with PLG molecules induced a small increase of muscle differentiation, as compared to IOX2 and FG-4592 pre-treatment.

### Structure of the tested substituted diacyl-hydrazine compounds:

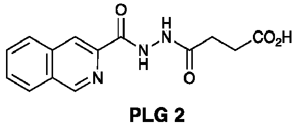

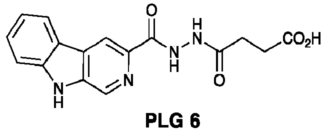

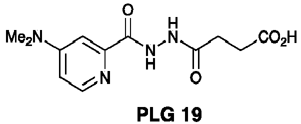

and their corresponding methyl esters:

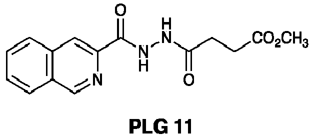

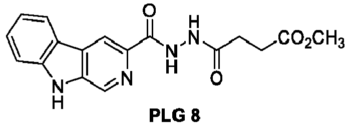

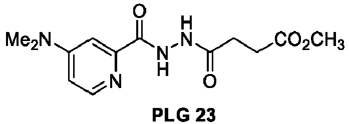

### METHODS

### Cell culture

C2C12 murine myoblasts (Merck) were cultured in growth medium (GM) made of Dulbecco's modified Eagle's medium (DMEM, Merck) with high glucose concentration (4.5 g/L) supplemented with 10% (v/v) fetal bovine serum (FBS, Merck), 2 mM glutamine (Merck), penicillin/streptomycin 1X (Euroclone) (GM) at 37°C in a 5% CO₂ and 95% air-humidified atmosphere. Cells were treated for 24 hours with PHDs inhibitors (PLG) at 37°C in a 5% CO₂ / 21% O₂. In order to induce muscle differentiation, 75 x 10³ C2C12 murine myoblasts were seeded in 35 mm culture plates, cultured in GM for 24 hours, and then switched to a differentiation medium (DM) containing DMEM supplemented with 2% (v/v) horse serum (HS) (Merck) [27]. The DM was changed every day for 7 days. Cells were analyzed at 24 hours post-treatment (PT), at 3^{rd} and 7^{th} days of skeletal muscle differentiation. These cells were tested for mycoplasma contamination before experiments and they are negative.

### Determination of the EC₅₀ for the PLG inhibitors

The EC₅₀ was calculated using a Dual-Glo® Luciferase Assay System kit (Promega) that allows a high-throughput analysis of mammalian cells containing the Firefly (Luciferase-pcDNA3, ODD-Luciferase-pcDNA3) and Renilla (pRL-CMV) luciferase reporters. Briefly, 5 x 10³ cells per well were seeded in a 96-well plate, and then transfected for 24 hours with a Luciferase-pcDNA3 or an ODD-Luciferase-pcDNA3 (80 ng), containing the firefly gene reporter. A co-transfection with pRL-CMV (8 ng), containing the renilla gene reporter, was performed as an internal control, according to the ViaFect Transfection Reagent® protocol (Promega). After transfection, cells were treated with 50, 100, 250, 500 and 1000 □M of PLG2 or PLG19 or PLG6, PLG8 or PLG11 or PLG23 for 24 hours, and then an equal volume of Dual-Glo® Luciferase Reagent was added to each well, incubated at room temperature for 30 minutes, and then analyzed with a Varioskan™ LUX Multimode Microplate Reader (Thermo Fisher Scientific). Then, an equal volume of Dual-Glo® Stop & Glo® Reagent was added to each well in order to quench the luminescence from the firefly reaction and to provide the substrate for the renilla luciferase. The mixture was incubated for 30 minutes before analysis. The EC₅₀ values were calculated by linear regression.

### Cell viability assay

RealTime-Glo™ kits MT Cell Viability Assay (Promega) was used to investigate the effects of PLG treatment on cell viability. Briefly, 5 x 10³ C2C12 murine myoblasts were seeded in a 96 well plate and incubated for 24 hours (T0) with MT Cell Viability Substrate (1:1000 dilution) and NanoLuc® Enzyme (1:1000 dilution). Then, each PLG inhibitor was added at its EC50 to the cell cultures and incubated for other 24 hours before luminescence analysis.

### Cell toxicity assay

To assess PHDs inhibitors cytotoxicity, 5 x 10³ C2C12 murine myoblasts were seeded in a 96-well plate. Each PLG inhibitor was added at its EC₅₀ to C2Cl2 for 24 hours before analysis with a CellTox™ Cytotoxicity Assay (Promega). Briefly, 100 µl of CellTox Buffer containing CellTox Green Dye (1:500 dilution) was added to each well and incubated at room temperature for 15 minutes before fluorescence analysis (excitation wavelength of 485-500 nm and emission filter of 520-530 nm).

### RNA extraction and gene expression by Real-Time Quantitative PCR (qPCR)

Total RNA was isolated using ReliaPrepTM RNA Miniprep Systems (Promega), and reverse transcribed to cDNA using the iScript cDNA synthesis kit (BioRad), according to the Manufacturer's instructions. Real-Time Quantitative PCR was performed on 10 ng of cDNA template, 0.2 µM primers, and GoTaq® qPCR Master Mix (Promega) in 20 µl final volume using a StepOnePlus® Real-Time PCR System (Applied Biosystem). The following primers were used: VEGF forward 5'-AAAAACGAAAGCGCA-3' (SEQ ID NO:17) and reverse 5'-TTTCTCCGCTCTGAA-3' (SEQ ID NO:18); PHD2 forward 5'-CTGTGGAACAGCCCTTTTT-3' (SEQ ID NO:19) and reverse 5'-CGAGTCTCTCTGCGAATCCT-3' (SEQ ID NO:20); MYOG forward 5'-AGCCACACTGAGGGA-3' (SEQ ID NO:21) and reverse 5'-GTTGAGGGAGCTGAG-3' (SEQ ID NO:22); MHC forward 5'-TGGAGCAGGAGGAATACAAG-3' (SEQ ID NO:23) and reverse 5'-GCATAGTGGATGAGGGAGAA-3' (SEQ ID NO:24); RPL13 forward 5'-CTCGGCCGTTCCTGTAT-3' (SEQ ID NO:29) and reverse 5'-GTGGAAGTGGGGCTTCAGTA-3' (SEQ ID NO:30). RPL13 was used as the housekeeping gene. The amplification program consisted in an initial denaturation at 95°C for 3 minutes, followed by 40 cycles of 5 seconds each at 95°C and 30 seconds at 57°C; WNT7a was amplified at 53°C. Relative quantification of target genes was performed in triplicate and calculated by the equation 2-ΔΔCt.

### Total proteins extraction and isolation of the nucleus compartment

For total proteins extraction, 7.5 x 10⁴ cells were seeded and harvested at 7th days of skeletal muscle differentiation, as previously described. Cells were collected after enzymatic digestion with trypsin (Merck), lysed by sonication and centrifuged at 800 xg for 10 minutes. Supernatant was used to determine total protein content with the BCA Protein Assay Kit (Pierce), according to the manufacturer's instructions. For nuclei separation, 5 x 10⁶ cells were collected after enzymatic digestion with trypsin (Merck), centrifuged at the maximum speed, resuspended in 400 µl of Buffer A (10 mM KCl, 0.1 mM EDTA, 1 mM DTT, 10 mM Hepes, pH 7.9), containing protease and phosphatase inhibitors cocktail (Merck), and incubated on ice for 20 minutes. Then, 10% NP40 was added to the cell suspension, mixed for 40 seconds, and centrifuged 40 seconds at the maximum speed. After centrifugation, the pellet was resuspended in 80 µl of Buffer C (0.4 M NaCl, 1 mM EDTA, 1 mM DTT, 20 mM Hepes, pH 7.9), containing a protease and phosphatase inhibitors cocktail, and then mixed for 30 minutes at 4°C at the maximum speed. Finally, the nuclear suspension was centrifuged for 5 minutes at 4°C at the maximum speed, collected and transferred in a new tube. The total protein content was determined with the BCA Protein Assay Kit (Pierce), according to the manufacturer's instructions.

### Western blot analyses

Proteins were denaturated by boiling for 10 minutes in sample buffer (0.6 g/100 mL Tris, 2 g/100 mL SDS, 10% glycerol, 1% 2-mercaptoethanol, pH 6.8) and loaded into 10% SDS-PAGE gel, then transferred onto a nitrocellulose membrane (Trans-blot, Bio-Rad Laboratories) by electroblotting. Nitrocellulose membranes were incubated with a blocking solution containing 5% (w/v) non-fat dry milk or 5% (w/v) BSA (Merck) in Tris-buffer saline with 0.1% Tween® 20 (TBS-T) for 1 hour. Blots were incubated for 2 hours at room temperature with the following primary antibodies: rabbit monoclonal anti-HIF-la, 1:1000 dilution (clone D2U3T, Cell Signaling Technology), goat polyclonal anti-Lamin A/C, 1:1000 dilution (clone N-18, Santa Cruz Biotechnology), mouse monoclonal anti-MyoD1, 1:1000 dilution (clone 5.2F, Abcam), mouse monoclonal anti-myosin (skeletal, fast), 1:1000 dilution (clone MY-32, Merck) and rabbit polyclonal anti-EE1a, 1:1000 dilution (Cell Signaling Technology). Membranes were washed three times for 10 minutes with TBS-T, and then incubated with the appropriate anti-mouse, anti-rabbit, or anti-goat HRP-conjugated secondary antibodies (Dako, Agilent Technologies), 1:2000 dilution, for 1 hour at room temperature. After three washes for 10 minutes with TBS-T, the immunoreactive bands were visualized using the enhanced chemiluminescence detection kit reagents (ECL Advance, GeHealthcare), according to the manufacturer's instructions.

### Immunofluorescence staining

After differentiation for 7 days in DM, cells were washed with phosphate saline buffer (PBS) and fixed for 15 minutes in 4% (w/v) paraformaldehyde at room temperature. For permeabilization and blocking, cells were incubated for 1 hour in the presence of PBS 0.1% (v/v) Triton X-100 (TX-100, Merck) and 5% (w/v) FBS (Merck) at room temperature. Then, cells were incubated for 2 hours at room temperature with mouse monoclonal anti-Myosin (Skeletal, fast) (clone MY-32, Merck), diluted 1:200 in PBS 0.1% (v/v) Triton X-100 (TX-100) and 5% (w/v) FBS. After incubation, cells were washed three times in PBS and incubated for 1 hour at room temperature with an anti-mouse FITC-conjugated secondary antibody (Jackson ImmunoResearch), diluted 1:200. Cell nuclei were counterstained with Hoechst 33342 (1:500 dilution, Merck). Myogenesis was assessed by measuring the myotube area and the number of myonuclei per myotube using a fluorescent microscope (Olympus TH4-200) equipped with an acquisition camera. To quantify both differentiation and fusion indexes, ten fields were chosen randomly and, for each field, a minimum of one hundred myosin-positive myotubes with more than two myonuclei were measured using the ImageJ v1.49o software. The area and the number of nuclei per myotube was the mean of ten measurements averaged from three different experiments.

### RESULTS

### EC₅₀ calculation of PLG inhibitors

In order to find new prolyl-hydroxylases inhibitors able to mimic the effects observed upon chemical and physical hypoxia on myogenesis, six new molecules were synthetized and characterized: three acid form (PLG2, PLG6 and PLG19) and their esters (PLG11, PLG8 and PLG23). The EC₅₀ of each compound was determined by transiently co-transfecting C2C12 myoblasts with the ODD-luciferase-pcDNA3 plasmid, containing the Oxygen Dependent Domain (ODD) of HIF-1α, and with the pRL-CMV plasmid, as the internal control (Figure 12, panel A). Then, transfected cells were treated for 24 hours with each PLG inhibitors at five different concentrations (50, 100, 250, 500 and 1000 µM) under normoxic conditions for 24 hours, and the emitted luminescence was collated to untreated controls. Results showed that the EC₅₀ of PLG6 and PLG19 and PLG23 was undetectable, while the EC₅₀ of PLG8, PLG11 and PLG 2 was calculated to be 250 µM respectively (Figure 12, panel B). Then, cells were treated with PLG8, PLG11 and PLG 2 at their EC₅₀ concentration for 24 hours and the effects on cell viability and toxicity were investigated. Results revealed a 60%, 17% and 20 % decrease in the proliferation rate induced by PLG8, PLG11 and PLG2, respectively (Figure 12, panel C), which was not due to a toxic effect of the treatment (Figure 12, panel D). Furthermore, the activation of HIF-1α and its main target genes were evaluated. Unlike the untreated control, PLG8 PLG11 and PLG2 treatments promoted a 1.5-, 1.5- and 1.3-fold protein increase of HIF-1α nuclear translocation, respectively (Figure 13, panel A). To confirm the activation of HIF-1α pathway, the gene expression of VEGF and PHD2 was evaluated. Data showed that PLG8, PLG11 and PLG2 inhibitors induced a 1.6-, 1.2- and 1.1-fold enhancement of VEGF (Figure 13, panel B). Along this line, PLG8 and PLG11 treatment caused a 1.8- and 1.3-fold increase of PHD2, while no significant changes were observed for PLG2 (Figure 13, panel C).

### Effects of PLG inhibitors on myogenesis

To assess whether PLG treatment would affect myogenesis, murine myoblasts C2C12 were pre-treated with 250 µM PLG8, PLG11 and PLG2 in GM for 24 hours, and then induced to differentiate for seven days by switching them to DM without the PHDs inhibitors. At the end of the differentiation process, an extensive formation of myosin heavy chain (MHC) positive myotubes was observed in both pre-treated and untreated cells (Figure 14, panel A). Quantitative evaluation of the differentiation parameters revealed no significant changes in the fusion indexes, whereas a 1.15-, 1.2- and 1.15-fold increase in the differentiation indexes could be observed in PLG8, PLG11 and PLG2 pre-treated myoblasts, respectively, as compared to untreated controls (Figure 14, panel B). Protein expression analyses revealed that PLG8, PLG11 and PLG2 induced a 1.4-, 1.3- and 1.2-fold increase, respectively, of the nuclear localization of the early differentiation marker, MyoD (Figure 14, panel C). Finally, gene expression analyses showed a 1.4-, 1.4- and 1.2-fold increase of Myogenin at day 3, and a 1.5-, 1.4 and 1.3-fold enhancement of MHC at day 7 of differentiation in PLG8, PLG11 and PLG2 pre-treated myoblasts, respectively (Figure 14, panel D and E).

### REFERENCES

1. Kuang, S., Kuroda, K., Le Grand, F. and Rudnicki, M. A. (2007). Cell 129, 999-1010.
2. Garcia-Prat, L., Sousa-Victor, P. and Munoz-Canoves, P. (2013). FEBS J 280, 4051-62.
3. Ambrosio, F., Kadi, F., Lexell, J., Fitzgerald, G. K., Boninger, M. L. and Huard, J. (2009). Am J Phys Med Rehabil 88, 145-55.
4. Le Grand, F., Jones, A. E., Seale, V., Scime, A. and Rudnicki, M. A. (2009). Cell Stem Cell 4, 535-47.
5. Gough, N. R. (2012). Sci Signal 5, eg2.
6. Tanaka, S., Terada, K. and Nohno, T. (2011). J Mol Signal 6, 12.
7. Bentzinger, C. F., von Maltzahn, J., Dumont, N. A., Stark, D. A., Wang, Y. X., Nhan, K., Frenette, J., Cornelison, D. D. and Rudnicki, M. A. (2014). J Cell Biol 205,97-111.
8. von Maltzahn, J., Bentzinger, C. F. and Rudnicki, M. A. (2011). Nat Cell Biol 14, 186-91.
9. Maxwell, P. H. and Eckardt, K. U. (2016). Nat Rev Nephrol 12, 157-68.
10. von Maltzahn, J., Zinoviev, R., Chang, N. C., Bentzinger, C. F. and Rudnicki, M. A. (2013). Nat Commun 4, 2869.
11. Hubbi, M. E. and Semenza, G. L. (2015). Am J Physiol Cell Physiol 309, C775-82.
12. Krock, B. L., Skuli, N. and Simon, M. C. (2011). Genes Cancer 2, 1117-33.
13. Di Carlo, A., De Mori, R., Martelli, F., Pompilio, G., Capogrossi, M. C. and Germani, A. (2004). J Biol Chem 279, 16332-8.
14. Majmundar, A. J., Lee, D. S., Skuli, N., Mesquita, R. C., Kim, M. N., Yodh, A. G., Nguyen-McCarty, M., Li, B. and Simon, M. C. (2015). 142, 2405-12.
15. Clarke, L. and van der Kooy, D. (2009). LStem Cells 27, 1879-86.
16. Eliasson, P. and Jonsson, J. I. (2010). J Cell Physiol 222, 17-22.
17. Gustafsson, M. V., Zheng, X., Pereira, T., Gradin, K., Jin, S., Lundkvist, J., Ruas, J. L., Poellinger, L., Lendahl, U. and Bondesson, M. (2005). Dev Cell 9, 617-28.
18. Mohyeldin, A., Garzon-Muvdi, T. and Quinones-Hinojosa, A. (2010). Cell Stem Cell 7, 150-61.
19. Liu, W., Wen, Y., Bi, P., Lai, X., Liu, X. S., Liu, X. and Kuang, S. (2012). Development 139, 2857-65.
20. Dengler, V. L., Galbraith, M. and Espinosa, J. M. (2014). Crit Rev Biochem Mol Biol 49, 1-15.
21. Wang, G. L., Jiang, B. H., Rue, E. A. and Semenza, G. L. (1995). H Proc Natl Acad Sci USA 92, 5510-4.
22. Yang, X., Yang, S., Wang, C. and Kuang, S. (2017). J Biol Chem 292, 5981-5991.
23. Xie, L., Yin, A., Nichenko, A. S., Beedle, A. M., Call, J. A. and Yin, H. (2018). J Clin Invest 128(6), 2339-2355.
24. Cirillo, F., Resmini, G., Ghiroldi, A., Piccoli, M., Bergante, S., Tettamanti, G. and Anastasia, L. (2017). FASEB J 31, 2146-2156.
25. Hoppe, G., Yoon, S., Gopalan, B., Savage, A. R., Brown, R., Case, K., Vasanji, A., Chan, E. R., Silver, R. B. and Sears, J. E. (2016). Proc Natl Acad Sci U S A 113, E2516-25.
26. Reano, S., Angelino, E., Ferrara, M., Malacarne, V., Sustova, H., Sabry, O., Agosti, E., Clerici, S., Ruozi, G., Zentilin, L. et al. (2017). Stem Cells 35, 1733-1746.
27. Scaringi, R., Piccoli, M., Papini, N., Cirillo, F., Conforti, E., Bergante, S., Tringali, C., Garatti, A., Gelfi, C., Venerando, B. et al. (2013). J Biol Chem 288, 3153-62.
28. Yeoh, K.K, Chan, M.C., Thalhammera, A., Demetriadesa, M., Chowdhurya, R., Tianb, Y., Stolzeb, I., McNeilla, L.A., Leec, M.K., Woona, E.C.Y., Mackeena, M.M., Kawamuraa, A., Ratcliffeb, P.J., Mecinovića, J., and Schofielda, C.J. (2013). Org Biomol Chem 11(5): 732-745.

## Claims

1. Proline hydroxylase inhibitor activating hypoxia inducible factor-1α, upregulating WNT7a expression and chelating iron, for use in the skeletal muscle regeneration.

2. Proline hydroxylase inhibitor for use according to claim 1, wherein the skeletal muscle regeneration occurs after physical or chemical hypoxic insult.

3. Proline hydroxylase inhibitor for use according to claim 1 or 2, for use in the treatment of muscular atrophy, muscular dystrophy, sarcopenia and myopathies.

4. Proline hydroxylase inhibitor for use according to anyone of claims 1 to 3, selected among the following groups:
i) an α-ketoglutarate analogue selected from the group consisting of: 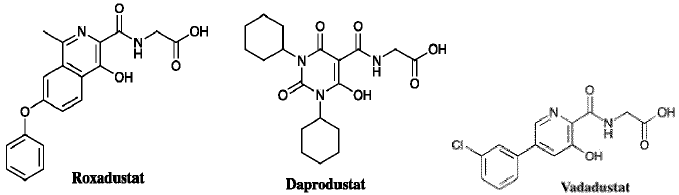 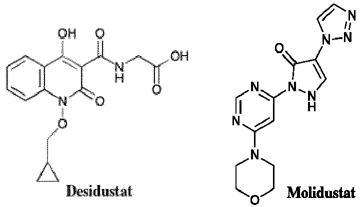 esters or salts thereof;
ii) a substituted diacyl-hydrazine compound **characterized by** the general formula (I): 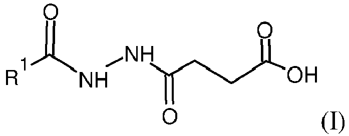 wherein R₁ is selected between isoquinoline 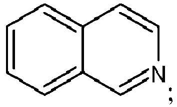 and a nitrogen aromatic ring of formula (II): 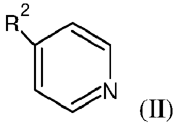 wherein
R₂ is an amine alkyl group selected from the group consisting of NHR₃, NR₃R₄, wherein R₃ and R₄ ugual or different each other, represents a linear or branched C₁-C₄ alkyl group;
or R₂, jointly with the C atom to which it is bound, forms a condensed monocycle or bicycle with 3-8 terminals, which contains from 1 to 2 nitrogen atoms, said condensed cycle being selected from the group consisting of: 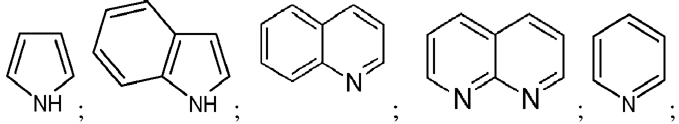 their esters or salts.

5. Proline hydroxylase inhibitor for use according to claim 4, wherein the substituted diacyl-hydrazine compounds of formula (I) are selected in the group comprising between: 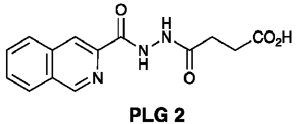 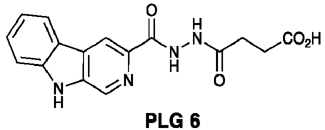 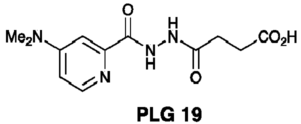 and the corresponding methyl esters thereof:

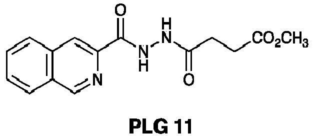

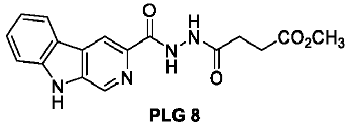

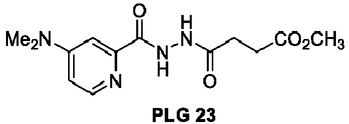

6. Proline hydroxylase inhibitor for use according to anyone of claims 1 to 5, to be administered by systemic or topical route.

7. Proline hydroxylase inhibitor for use according to anyone of claims 1 to 6, in a dosage range between 0.5 mg/kg and 200 mg/kg, preferably between 1 mg/kg to 100 mg/kg, more preferably between 2 mg/kg to 70 mg/kg.

8. Pharmaceutical composition comprising a proline hydroxylase inhibitor activating hypoxia inducible factor-1α and upregulating WNT7a expression as active ingredient, together with a pharmaceutically acceptable additives and/or excipients, for use in the skeletal muscle regeneration.

9. Pharmaceutical composition for use according to claim 8, wherein the skeletal muscle regeneration occurs after physical or chemical hypoxic insult.

10. Pharmaceutical composition according to claim 8 or 9, for use in the treatment of muscle atrophy, muscular dystrophy, sarcopenia and myopathies.

11. Pharmaceutical composition for use according to anyone of claims 8-10, wherein the proline hydroxylase inhibitor is selected among the following groups:
i) an analogue of α-ketoglutarate selected from the group consisting of i) an α-ketoglutarate analogue selected from the group consisting of: 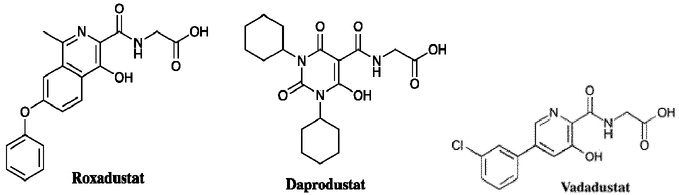 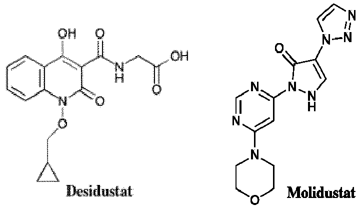 esters or salts thereof;
ii) a substituted diacyl-hydrazine compound **characterized by** the general formula (I): 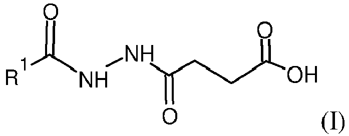 wherein R₁ is selected between isoquinoline 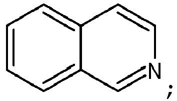 and a nitrogen aromatic ring of formula (II): 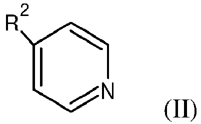
wherein R₂ is an amine alkyl group selected from the group consisting of NHR₃, NR₃R₄, wherein R₃ and R₄ ugual or different each other, represents a linear or branched C₁-C₄ alkyl group;
or R₂, jointly with the C atom to which it is bound, forms a condensed monocycle or bicycle with 3-8 terminals, which contains from 1 to 2 nitrogen atoms, said condensed cycle being selected from the group consisting of: 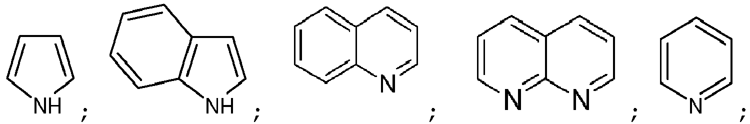 their esters or salts.

12. Pharmaceutical composition for use according to claim 11, wherein the substituted diacyl-hydrazine compounds of formula (I) are selected in the group comprising: 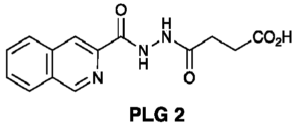 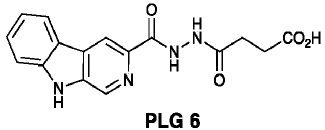 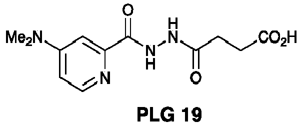 and the corresponding methyl esters thereof:

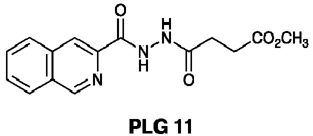

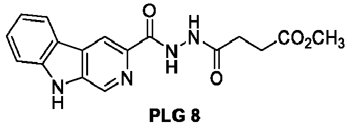

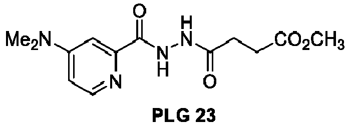

13. Pharmaceutical composition for use according to anyone of claims 8-12, for systemic or topical administration.

14. Pharmaceutical composition for topical administration according to claim 13, in the form of a transdermal patch, reservoir or matrix system.

15. Pharmaceutical composition for topical administration according to claim 13, in the form of liposomes, microparticles or microcapsules.
